# EUROPEAN PATENT APPLICATION

(11) **EP 3 708 664 A1**
(43) Date of publication of application: **16.09.2020**
(21) Application number: 18875341.2
(22) Date of filing: 08.11.2018
(51) Int. Cl.: C12N 15/11, A61K 31/7088, A61K 31/7115, A61K 48/00, A61P 31/04, A61P 31/10, A61P 31/12, A61P 33/00, A61P 35/00, A61P 37/04

(54) **METHOD FOR mRNA STABILIZATION**

(30) Priority: 09.11.2017 US 201762583691 P
(71) Applicant: The University Of Tokyo, Tokyo 113-8654 (JP); Kawasaki Institute of Industrial Promotion, Kawasaki-shi, Kanagawa 212-0013 (JP)
(72) Inventor: UCHIDA, Satoshi, Tokyo 113-8654 (JP); YOSHINAGA, Naoto, Tokyo 113-8654 (JP); CHO, Eol, Tokyo 113-8654 (JP); KOJI, Kyoko, Tokyo 113-8654 (JP)
(74) Representative: J A Kemp LLP
(86) International application number: PCT/JP2018/041493
(87) International publication number: WO 2019/093423

(57) **Abstract**

A complex of mRNA encoding a target gene with RNA oligomers, wherein the RNA oligomers are at least two kinds of RNA oligomers comprising RNA sequences of (a) or (b), the at least two kinds of RNA oligomers are hybridizable respectively with sequences, said sequences being different from each other, in the mRNA sequence, and the complex is formed when a first sequence of the RNA oligomers hybridizes with different mRNA from the second sequence of the RNA oligomers: (a) an RNA sequence which comprises a first sequence consisting of 12-40 bases and being complementary to the mRNA sequence, a linker sequence consisting of 0-100 bases and a sequence which is the same as the first sequence, in this order; and (b) an RNA sequence which comprises a first sequence, which has a 90% or more identity with a sequence consisting of 12-40 bases and being complementary to the mRNA sequence and is hybridizable with mRNA, a linker sequence consisting of 0-100 bases and a second sequence which is the same as the first sequence, in this order. Thus, a novel method for mRNA stabilization is provided.

## Description

### Technical Field

The present invention relates to a method for stabilizing an mRNA.

### Background Art

mRNA delivery has attracted attention as a method of supplying a therapeutic protein into a body safely and continuously. On the other hand, such mRNA delivery is greatly problematic in that enzymatic degradation of mRNA promptly occurs in a living body. To solve this issue, a method of protecting mRNA from degradation by using an mRNA carrier and a method of improving an mRNA molecule itself have been studied. However, since the action of RNA-degrading enzyme is extremely strong in a living body, further technical innovation has been desired.

Regarding the method of improving an mRNA molecule itself, a method of replacing some mRNA nucleotides with chemically modified nucleotides has been studied (for example, Non Patent Literature 1). To date, various chemically modified nucleotides have been comprehensively studied. However, chemically modified nucleotides capable of significantly improving the enzyme resistance of mRNA have not yet been reported. Moreover, since the efficiency of translating a protein from mRNA is often largely decreased by performing modification on the nucleotides, it has been difficult to perform chemical modification, as desired.

Patent Literature 1 discloses a polyion complex consisting of a cationic polymer and an mRNA, and the use of the polyion complex in mRNA delivery. In addition, Non Patent Literature 2 discloses the stability of a polymeric micelle-type mRNA carrier, evaluation of enzymatic degradation, and the effect of performing cholesterol modification on the polymer. However, these publications do not disclose a method for stabilizing an mRNA, by which the enzyme resistance of an mRNA can be improved in a living body, without largely decreasing the efficiency of translating a protein from the mRNA.

### Citation List

### Patent Literature

Patent Literature 1: WO 2015/121924 A

### Non Patent Literature

Non Patent Literature 1: Meis, J. E. and Chen, F. (2002) EPICENTRE Forum 9(1), 10
Non Patent Literature 2: Uchida, S., et al., Biomaterials (2016) 82, pp. 221-228.

### Summary of Invention

### Technical Problem

It is an object of the present invention to provide a novel method for stabilizing an mRNA.

### Solution to Problem

As a result of intensive studies, the present inventors have found that a complex consisting of an mRNA and RNA oligomers can be obtained by allowing at least two types of RNA oligomers comprising a first sequence complementary to the mRNA, a linker sequence, and a second sequence that is the same as the first sequence, to hybridize with the mRNA, and that this complex is capable of stabilizing the mRNA, thereby completing the present invention.

Specifically, the present invention is as follows.
[1-1] A complex consisting of an mRNA encoding a target gene and RNA oligomers, wherein
   the RNA oligomers are at least two types of RNA oligomers comprising the following RNA sequence (a) or (b),
   at least the two types of RNA oligomers are capable of hybridizing with each different sequence in the sequence of the mRNA, and
   a first sequence of the RNA oligomer hybridizes with an mRNA that is different from an mRNA for a second sequence of the RNA oligomer, so that the complex is formed (namely, mRNAs are crosslinked to one another by the RNA oligomers):
   (a) an RNA sequence comprising a first sequence of 12 to 40 nucleotides complementary to the mRNA sequence, a linker sequence of 0 to 100 nucleotides, and a second sequence that is the same as the first sequence, in this order; or
   (b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence that is the same as the first sequence, in this order.
[1-2] The complex according to the above [1-1], wherein the RNA oligomers are at least two types of RNA oligomers comprising the following RNA sequence (a) or (b):
   (a) an RNA sequence comprising a first sequence of 15 to 23 nucleotides complementary to the mRNA sequence, a linker sequence of 0 to 30 nucleotides, and a second sequence that is the same as the first sequence, in this order; or
   (b) an RNA sequence comprising a first sequence having an identity of 90% or more to the sequence of 15 to 23 nucleotides complementary to the mRNA sequence and also being capable of hybridizing with the mRNA, a linker sequence of 0 to 30 nucleotides, and a second sequence that is the same as the first sequence, in this order.
[1-3] The complex according to the above [1-1], wherein the RNA oligomers are at least two types of RNA oligomers comprising the following RNA sequence (a) or (b):
   (a) an RNA sequence comprising a first sequence of 17 nucleotides complementary to the mRNA sequence, a linker sequence of 10 nucleotides, and a second sequence that is the same as the first sequence, in this order; or
   (b) an RNA sequence comprising a first sequence having an identity of 90% or more to the sequence of 17 nucleotides complementary to the mRNA sequence and also being capable of hybridizing with the mRNA, a linker sequence of 10 nucleotides, and a second sequence that is the same as the first sequence, in this order.
[1-4] The complex according to any one of the above [1-1] to [1-3], wherein the nucleotides of the linker sequence are all adenines.
[1-5] The complex according to any one of the above [1-1] to [1-4], wherein at least the two types of RNA oligomers are 2 to 8 types of RNA oligomers.
[1-6] The complex according to any one of the above [1-1] to [1-5], which comprises the mRNA and at least the two types of RNA oligomers, such that the concentration of the mRNA becomes 1 µg/mL to 10,000 µg/mL, and the molar ratio between the mRNA and each RNA oligomer of at least the two types of RNA oligomers becomes 1 : 0.05 to 0.05 : 1.
[1-7] A pharmaceutical composition comprising the complex according to any one of the above [1-1] to [1-6].
[2-1] A method for stabilizing an mRNA, comprising forming a complex consisting of an mRNA encoding a target gene and at least two types of RNA oligomers comprising the following RNA sequence (a) or (b), wherein
   at least the two types of RNA oligomers are capable of hybridizing with each different sequence in the sequence of the mRNA, and
   in the complex, a first sequence of the RNA oligomer hybridizes with an mRNA that is different from an mRNA for a second sequence of the RNA oligomer:
   (a) an RNA sequence comprising a first sequence of 12 to 40 nucleotides complementary to the mRNA sequence, a linker sequence of 0 to 100 nucleotides, and a second sequence that is the same as the first sequence, in this order; or
   (b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence that is the same as the first sequence, in this order.
[2-2] The method according to the above [2-1], wherein the RNA oligomers are at least two types of RNA oligomers comprising the following RNA sequence (a) or (b):
   (a) an RNA sequence comprising a first sequence of 15 to 23 nucleotides complementary to the mRNA sequence, a linker sequence of 0 to 30 nucleotides, and a second sequence that is the same as the first sequence, in this order; or
   (b) an RNA sequence comprising a first sequence having an identity of 90% or more to the sequence of 15 to 23 nucleotides complementary to the mRNA sequence and also being capable of hybridizing with the mRNA, a linker sequence of 0 to 30 nucleotides, and a second sequence that is the same as the first sequence, in this order.
[2-3] The method according to the above [2-1], wherein the RNA oligomers are at least two types of RNA oligomers comprising the following RNA sequence (a) or (b):
   (a) an RNA sequence comprising a first sequence of 17 nucleotides complementary to the mRNA sequence, a linker sequence of 10 nucleotides, and a second sequence that is the same as the first sequence, in this order; or
   (b) an RNA sequence comprising a first sequence having an identity of 90% or more to the sequence of 17 nucleotides complementary to the mRNA sequence and also being capable of hybridizing with the mRNA, a linker sequence of 10 nucleotides, and a second sequence that is the same as the first sequence, in this order.
[2-4] The method according to any one of the above [2-1] to [2-3], wherein the nucleotides of the linker sequence are all adenines.
[2-5] The method according to any one of the above [2-1] to [2-4], wherein at least the two types of RNA oligomers are 2 to 8 types of RNA oligomers.
[2-6] The method according to any one of the above [2-1] to [2-5], which comprises the mRNA and at least the two types of RNA oligomers, such that the concentration of the mRNA becomes 1 µg/mL to 10,000 µg/mL, and the molar ratio between the mRNA and each RNA oligomer of at least the two types of RNA oligomers becomes 1 : 0.05 to 0.05 : 1.
[3-1] A complex consisting of an mRNA encoding a target gene and an RNA oligomer, wherein
   the RNA oligomer is at least one type of RNA oligomer comprising the following RNA sequence (a) or (b), and
   a first sequence of the RNA oligomer hybridizes with an mRNA that is different from an mRNA for a second sequence of the RNA oligomer, so that the complex is formed (namely, mRNAs are crosslinked to one another by the RNA oligomers):
   (a) an RNA sequence comprising a first sequence of 12 to 40 nucleotides complementary to the mRNA sequence, a linker sequence of 0 to 100 nucleotides, and a second sequence that is a sequence of 12 to 40 nucleotides complementary to the mRNA sequence and is different from the first sequence, in this order; or
   (b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence having an identity of 90% or more to the sequence of 12 to 40 nucleotides complementary to the mRNA sequence, being capable of hybridizing with the mRNA, and being different from the first sequence, in this order.
[3-2] The complex according to the above [3-1], wherein the RNA oligomer is at least one type of RNA oligomer comprising the following RNA sequence (a) or (b):
   (a) an RNA sequence comprising a first sequence of 15 to 23 nucleotides complementary to the mRNA sequence, a linker sequence of 0 to 30 nucleotides, and a second sequence that is a sequence of 15 to 23 nucleotides complementary to the mRNA sequence and is different from the first sequence, in this order; or
   (b) an RNA sequence comprising a first sequence having an identity of 90% or more to the sequence of 15 to 23 nucleotides complementary to the mRNA sequence and also being capable of hybridizing with the mRNA, a linker sequence of 0 to 30 nucleotides, and a second sequence having an identity of 90% or more to the sequence of 15 to 23 nucleotides complementary to the mRNA sequence, being capable of hybridizing with the mRNA, and being different from the first sequence, in this order.
[3-3] The complex according to the above [3-1], wherein the RNA oligomer is at least one type of RNA oligomer comprising the following RNA sequence (a) or (b):
   (a) an RNA sequence comprising a first sequence of 17 nucleotides complementary to the mRNA sequence, a linker sequence of 10 nucleotides, and a second sequence that is a sequence of 17 nucleotides complementary to the mRNA sequence and is different from the first sequence, in this order; or
   (b) an RNA sequence comprising a first sequence having an identity of 90% or more to the sequence of 17 nucleotides complementary to the mRNA sequence and also being capable of hybridizing with the mRNA, a linker sequence of 10 nucleotides, and a second sequence having an identity of 90% or more to the sequence of 17 nucleotides complementary to the mRNA sequence, being capable of hybridizing with the mRNA, and being different from the first sequence, in this order.
[3-4] The complex according to any one of the above [3-1] to [3-3], wherein the nucleotides of the linker sequence are all adenines.
[3-5] The complex according to any one of the above [3-1] to [3-4], wherein at least the one type of RNA oligomer is 2 to 8 types of RNA oligomers.
[3-6] The complex according to any one of the above [3-1] to [3-5], which comprises the mRNA and at least the one type of RNA oligomer, such that the concentration of the mRNA becomes 1 µg/mL to 10,000 µg/mL, and the molar ratio between the mRNA and each RNA oligomer of at least the one type of RNA oligomer becomes 1 : 0.05 to 0.05 : 1.
[3-7] A pharmaceutical composition comprising the complex according to any one of the above [3-1] to [3-6].
[4-1] A method for stabilizing an mRNA, comprising forming a complex consisting of an mRNA encoding a target gene and at least one type of RNA oligomer comprising the following RNA sequence (a) or (b), wherein
   in the complex, a first sequence of the RNA oligomer hybridizes with an mRNA that is different from an mRNA for a second sequence of the RNA oligomer:
   (a) an RNA sequence comprising a first sequence of 12 to 40 nucleotides complementary to the mRNA sequence, a linker sequence of 0 to 100 nucleotides, and a second sequence that is a sequence of 12 to 40 nucleotides complementary to the mRNA sequence and is different from the first sequence, in this order; or
   (b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence having an identity of 90% or more to the sequence of 12 to 40 nucleotides complementary to the mRNA sequence, being capable of hybridizing with the mRNA, and being different from the first sequence, in this order.
[4-2] The method according to the above [4-1], wherein the RNA oligomer is at least one type of RNA oligomer comprising the following RNA sequence (a) or (b):
   (a) an RNA sequence comprising a first sequence of 15 to 23 nucleotides complementary to the mRNA sequence, a linker sequence of 0 to 30 nucleotides, and a second sequence that is a sequence of 15 to 23 nucleotides complementary to the mRNA sequence and is different from the first sequence, in this order; or
   (b) an RNA sequence comprising a first sequence having an identity of 90% or more to the sequence of 15 to 23 nucleotides complementary to the mRNA sequence and also being capable of hybridizing with the mRNA, a linker sequence of 0 to 30 nucleotides, and a second sequence having an identity of 90% or more to the sequence of 15 to 23 nucleotides complementary to the mRNA sequence, being capable of hybridizing with the mRNA, and being different from the first sequence, in this order.
[4-3] The method according to the above [4-1], wherein the RNA oligomer is at least one type of RNA oligomer comprising the following RNA sequence (a) or (b):
   (a) an RNA sequence comprising a first sequence of 17 nucleotides complementary to the mRNA sequence, a linker sequence of 10 nucleotides, and a second sequence that is a sequence of 17 nucleotides complementary to the mRNA sequence and is different from the first sequence, in this order; or
   (b) an RNA sequence comprising a first sequence having an identity of 90% or more to the sequence of 17 nucleotides complementary to the mRNA sequence and also being capable of hybridizing with the mRNA, a linker sequence of 10 nucleotides, and a second sequence having an identity of 90% or more to the sequence of 17 nucleotides complementary to the mRNA sequence, being capable of hybridizing with the mRNA, and being different from the first sequence, in this order.
[4-4] The method according to any one of the above [4-1] to [4-3], wherein the nucleotides of the linker sequence are all adenines.
[4-5] The method according to any one of the above [4-1] to [4-4], wherein at least the one type of RNA oligomer is 2 to 8 types of RNA oligomers.
[4-6] The method according to any one of the above [4-1] to [4-5], which comprises the mRNA and at least the one type of RNA oligomer, such that the concentration of the mRNA becomes 1 µg/mL to 10,000 µg/mL, and the molar ratio between the mRNA and each RNA oligomer of at least the one type of RNA oligomer becomes 1 : 0.05 to 0.05 : 1.
[5-1] An mRNA carrier, which loads therein the complex according to any one of the above [1-1] to [1-6] and [3-1] to [3-6].
[5-2] The mRNA carrier according to the above [5-1], wherein the carrier is a polymeric micelle or a lipidic mRNA carrier.
[5-3] A pharmaceutical composition comprising the mRNA carrier according to the above [5-1] or [5-2].

### Advantageous Effects of Invention

The present invention provides a novel method for stabilizing an mRNA. Preferably, in the present invention, an mRNA can be stabilized *in vivo,* while maintaining protein translation efficiency from the mRNA. More preferably, in the present invention, enzymatic degradation of an mRNA *in vivo* can be suppressed.

### Brief Description of Drawings

[Figure 1] Figure 1 is a conceptual view of an mRNA nano-assembly. (A) Formation of an mRNA nano-assembly; and (B) an RNA oligomer.
[Figure 2] Figure 2 is a view showing the mRNA nano-assemblies used in the Examples. (A) Types of mRNA nano-assemblies (only mRNA, mRNA + non-linker RNA oligomers, and mRNA + linker RNA oligomers); and (B) hybridization conditions.
[Figure 3] Figure 3 is a view showing the analysis of the physical properties of the mRNA nano-assemblies produced in the Examples. (A) The results of the analysis of size by FCS (Fluorescence Correlation Spectroscopy); (B) photographs of AFM (Atomic Force Microscopy).
[Figure 4] Figure 4 is a view showing the results of the enzyme resistance test of the mRNA nano-assemblies produced in the Examples and the analysis of the translation activity thereof in a cell-free system. (A) Enzyme resistance test; and (B) the analysis of translation activity in a cell-free system.
[Figure 5] Figure 5 is a view showing the uptake amount of the mRNA nano-assemblies produced in the Examples into cultured cells and the expression level of the mRNA in the cells. (A) Experimental procedures; (B) intracellular uptake amount; and (C) the expression level of the mRNA.
[Figure 6] Figure 6 shows the sequence of the mRNA of the Gaussia Luciferase (Gluc) produced in the Examples (SEQ ID NO: 2). The underlined portion indicates open reading frame.
[Figure 7] Figure 7 shows the coding sequence of the Gaussia Luciferase (Gluc) used in the Examples (SEQ ID NO: 1).
[Figure 8] Figure 8 is a view showing the results obtained by evaluating the particle diameters of the polymeric micelles (PMs) produced using PEG-PAsp(DET) in the Examples.
[Figure 9] Figure 9 is a view showing the results obtained by evaluating the particle diameters of the polymeric micelles (PMs) produced using PEG-PLys in the Examples.
[Figure 10] Figure 10 is a view showing the results of the enzyme resistance test performed, under 10% serum culture, on the mRNA nano-assembly produced using PEG-PAsp(DET) in the Examples.
[Figure 11] Figure 11 is a view showing the results of the enzyme resistance test performed, under 50% serum culture, on the mRNA nano-assembly produced using PEG-PLys in the Examples.
[Figure 12] Figure 12 shows the coding sequence of EGFP used in the Examples (SEQ ID NO: 21).
[Figure 13] Figure 13 is a view showing the results of the analysis of the size of the EGFP mRNA nano-assemblies produced in the Examples by FCS (Fluorescence Correlation Spectroscopy) (N = 5).
[Figure 14] Figure 14 is a view showing the results of the degrading enzyme resistance test of the EGFP mRNA nano-assemblies produced in the Examples in serum (N = 4).
[Figure 15] Figure 15 is a view showing the results of the analysis of the size of the EGFP mRNA nano-assemblies produced in the Examples according to AFM (Atomic Force Microscopy). (A) and (B) Unhybridized; and (C) and (D) Nano-assembly.
[Figure 16] Figure 16 is a view showing the results of the analysis of the size of the EGFP mRNA nano-assemblies produced in the Examples by FFF (Field forward fractionation). (A) Unhybridized; and (B) Nano-assembly.

### Description of Embodiments

Hereinafter, the present invention will be described in detail. The following embodiments are provided only for illustrative purpose of explaining the present invention. Thus, the present invention can be carried out in various forms unless it is deviated from the gist thereof. In addition, all publications cited in the present description, for example, prior art documents and patent documents such as published publications and patent publications, are incorporated herein by reference in their entirety. Moreover, the present description includes part or all of the contents as disclosed in the claims, description and drawings of U.S. Provisional Application No. 62/583,691 (filed on November 9, 2017), which is the priority document of the present application.

### 1. Summary

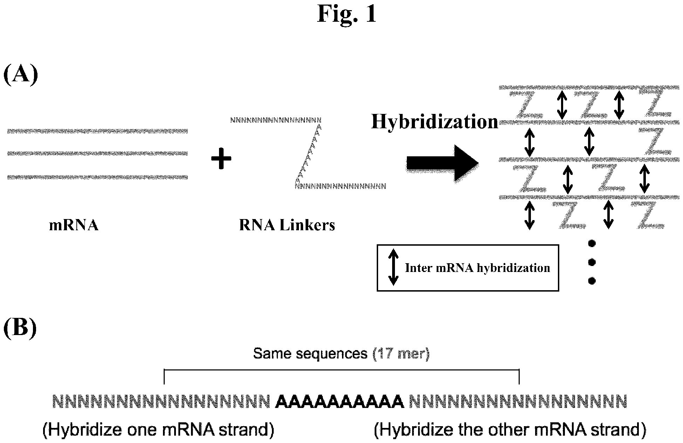

Herein, a complex consisting of an mRNA encoding a target gene and RNA oligomers (hereinafter also referred to as an "mRNA nano-assembly") is provided (Figure 1(A)). In some aspects, the RNA oligomer is an RNA sequence comprising a first sequence substantially complementary to the sequence of the mRNA, optionally, a linker sequence, and a second sequence that is the same as the first sequence, in this order (Figure 1(B)). At least two types of RNA oligomers are used. At least two types of RNA oligomers are used with respect to one type of mRNA, and the mRNA is allowed to hybridize with the RNA oligomers, so that an mRNA nano-assembly can be produced. In such an mRNA nano-assembly, mRNAs approach to one another by the action of the RNA oligomers, namely, by the crosslinking of the mRNAs by the RNA oligomers. Thus, the mRNAs approach to one another, so that hybridization is partially formed even among the mRNAs (Figure 1(A)).

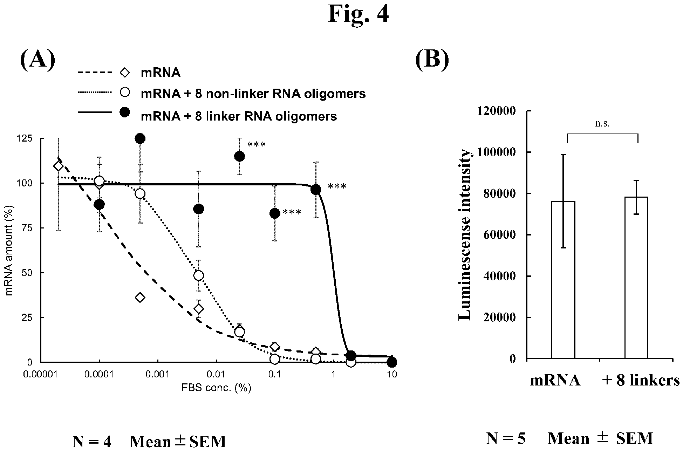

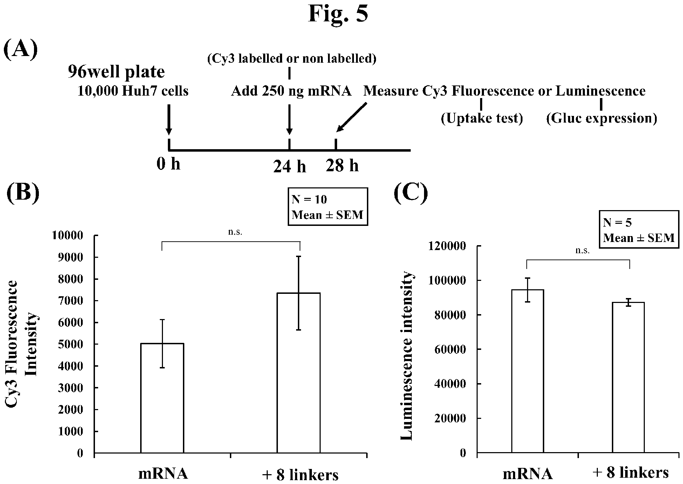

It is considered that the mRNA comprised in the mRNA nano-assembly is hardly recognized by degrading enzyme due to its steric hindrance, in comparison to a single-stranded mRNA. As a matter of fact, in the mRNA nano-assembly, enzyme resistance was significantly improved (Figure 4(A)). In addition, it was found that translation activity is kept in the mRNA nano-assembly, while the intracellular uptake efficiency of the mRNA nano-assembly is almost the same as that of the mRNA (Figure 4(B), and Figures 5(B) and (C)).

### 2. Complex

The present invention provides a complex consisting of an mRNA encoding a target gene and RNA oligomers, wherein
the RNA oligomers are at least two types of RNA oligomers comprising the following RNA sequence (a) or (b),
at least the two types of RNA oligomers are capable of hybridizing with each different sequence in the sequence of the mRNA, and
a first sequence of the RNA oligomer hybridizes with an mRNA that is different from an mRNA for a second sequence of the RNA oligomer, so that the complex is formed (namely, mRNAs are crosslinked to one another by the RNA oligomers):
(a) an RNA sequence comprising a first sequence of 12 to 40 nucleotides complementary to the mRNA sequence, a linker sequence of 0 to 100 nucleotides, and a second sequence that is the same as the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence that is the same as the first sequence, in this order.

Moreover, the present invention provides a complex consisting of an mRNA encoding a target gene and an RNA oligomer, wherein
the RNA oligomer is at least one type of RNA oligomer comprising the following RNA sequence (a) or (b), and
a first sequence of the RNA oligomer hybridizes with an mRNA that is different from an mRNA for a second sequence of the RNA oligomer, so that the complex is formed (namely, mRNAs are crosslinked to one another by the RNA oligomers):
(a) an RNA sequence comprising a first sequence of 12 to 40 nucleotides complementary to the mRNA sequence, a linker sequence of 0 to 100 nucleotides, and a second sequence that is a sequence of 12 to 40 nucleotides complementary to the mRNA sequence and is different from the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence having an identity of 90% or more to the sequence of 12 to 40 nucleotides complementary to the mRNA sequence, being capable of hybridizing with the mRNA, and being different from the first sequence, in this order.

A person skilled in the art could appropriately select a target gene, depending on purpose. Examples of the target gene may include a reporter gene, a growth factor gene, a cell growth factor gene, a cell growth suppressor gene, a cell death promoting factor gene, a cell death suppressor gene, a tumor suppressor gene, a transcription factor gene, a genome editing gene, and a vaccine antigen gene. For example, to a subject in need of proliferation of specific cells, a complex comprising an mRNA encoding a growth factor gene for the specific cells is administered, so that the disease or condition of the subject can be treated.

Examples of the reporter may include a luminescent protein and a fluorescent protein.

Examples of the growth factor may include an epidermal growth factor (EGF), an insulin-like growth factor (IGF), a nerve growth factor (NGF), a brain-derived neurotrophic factor (BDNF), a vascular endothelial growth factor (VEGF), a granulocyte colony stimulating factor (G-CSF), a granulocyte-macrophage colony stimulating factor (GM-CSF), a platelet-derived growth factor (PDGF), erythropoietin (EPO), thrombopoietin (TPO), a basic fibroblast growth factor (bFGF or FGF- 2), and a hepatocyte growth factor (HGF).

Examples of the cell growth suppressor may include p21, p17, p16, and p53.

Examples of the cell death promoting factor may include Smac/Diablo, an apoptosis inducing factor (AIF), HtrA2, Bad, Bim, Bax, p53, caspase 1, 2, 3, 4, 5, 6, 7, 8, 9 and 10 (e.g., caspases 2, 3, 6, 7, 8, 9 and 10, preferably caspase 3, 6 and 7), Fas ligand (FasL), tumor necrosis factor related apoptosis-inducing ligand (TRAIL), and FoxO1.

Examples of the cell death suppressor may include anti-apoptotic factors (e.g., FLIP, Mcl-1, Xiap, crmA, Bcl-2, and Bcl-xL).

Examples of the tumor suppressor gene may include p53, a retinoblastoma gene (Rb), an adenomatous polyposis coli gene (APC), a neurofibromatosis type 1 gene (NF1), a neurofibromatosis type 2 gene (NF2), WT1, VHL, BRCA1, BRCA2, CHEK2, maspin, p73, Smad4, MSH2, MLH1, PMS2, DCC, phosphatase tensin homolog (PTEN), SDHD, p16, p57^{Kip2}, PTC, TSC1, TSC2, EXT1, and EXT2.

Examples of the transcription factor may include Runt-related transcription factor 1 (Runx1), p53, c-fos, c-Jun, CREB, C/EBP, MyoD, c-Myc, c-Myb, Oct3/4, NF-κB, NF-AT, Mef-2, and an extracellular signal response factor (SRF).

Examples of the genome editing gene may include zinc finger nuclease (ZNF), transcription activator like effector nuclease (TALEN), and a clustered, regularly interspaced, short palindromic repeat (CRISPR)/CRISPR-associated (Cas) 9 gene.

Examples of the vaccine antigen gene may include a pathogen antigen and a tumor specific antigen.

The term "mRNA" means a messenger RNA, and the mRNA generally comprises a 5' untranslated region (5' UTR), a coding region, and a 3' untranslated region (3' UTR). In general, the mRNA further comprises a cap structure at the 5'-terminus (5' Cap) and a poly A sequence at the 3'-terminus.

The mRNA used herein may be any one of the following mRNAs.
(1) mRNA comprising 5' Cap, 5' UTR, a coding region, 3' UTR, and poly A in this order.
(2) mRNA comprising 5' Cap, 5' UTR, a coding region, and poly A in this order.
(3) mRNA comprising 5' UTR, a coding region, 3' UTR, and poly A in this order.
(4) mRNA comprising 5' UTR, a coding region, and poly A in this order.
(5) mRNA comprising 5' Cap, 5' UTR, a coding region, and 3' UTR in this order.
(6) mRNA comprising 5' Cap, 5' UTR, and a coding region in this order.
(7) mRNA comprising 5' UTR, a coding region, and 3' UTR in this order.
(8) mRNA comprising 5' UTR and a coding region in this order.

The mRNA encoding a target gene can be produced by transcribing a template DNA encoding the target gene under *in vitro* environment according to a known method. For example, the mRNA encoding a target gene can be produced according to the method described in Blood 108 (13) (2006) 4009-17. Specifically, a template DNA, in which a poly A/T strand is incorporated downstream of a protein coding sequence, is cleaved immediately downstream of the poly A/T strand, and is then subjected to *in vitro* transcription in a buffer solution containing translation enzymes, nucleosides and 5' Cap analogs. Thereafter, the mRNA is purified, so as to produce an mRNA encoding a target gene. A more specific method of preparing an mRNA is as described in the after-mentioned Examples.

In some aspects, chemical modification is not performed on the nucleotides of the mRNA itself. In this case, since the mRNA used here is almost the same as endogenous mRNA, it can be expected that the translation process is hardly impaired. In some other aspects, chemical modification is performed on the nucleotides of the mRNA itself. It has been known that chemical modification performed on the nucleotides of the mRNA itself, for example, can improve the enzyme resistance of the mRNA or can reduce immunogenicity. Examples of the chemically modified nucleotides of the mRNA itself may include methylated nucleotides (e.g., 5-methylcytosine), sulfur modified nucleotides (e.g., 2-thiouridine), pseudouridine, N1 methyl pseudouridine, and 5-methoxy-uridine.

In some aspects, the RNA oligomer is an RNA strand comprising the following sequence (a) or (b):
(a) an RNA sequence comprising a first sequence of 12 to 40 nucleotides complementary to the mRNA sequence, a linker sequence of 0 to 100 nucleotides, and a second sequence that is the same as the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence that is the same as the first sequence, in this order.

In some other aspects, the RNA oligomer is an RNA strand comprising the following sequence (a) or (b):
(a) an RNA sequence comprising a first sequence of 12 to 40 nucleotides complementary to the mRNA sequence, a linker sequence of 0 to 100 nucleotides, and a second sequence that is a sequence of 12 to 40 nucleotides complementary to the mRNA sequence and is different from the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence having an identity of 90% or more to the sequence of 12 to 40 nucleotides complementary to the mRNA sequence, being capable of hybridizing with the mRNA, and being different from the first sequence, in this order.

The first sequence and the second sequence of the RNA oligomer are set to be the same complementary sequences that hybridize with an identical sequence on one type of mRNA. The first and second sequences are used to crosslink between mRNAs of the same type, so as to form a complex. If the first and second sequences are able to form a complex, the first sequence and the second sequence each hybridize with an identical sequence on one type of RNA, although the first sequence and the second sequence are not necessarily the same sequences. However, the first and second sequence can also be constituted with complementary sequences capable of forming a complete match, mismatch, or bulge structure. Moreover, a chimeric sequence such as RNA/DNA can also be inserted therein. Furthermore, upon the designing of the RNA oligomer that crosslinks between one-type mRNAs, the sequences on the mRNA, with which the first sequence and the second sequence hybridize, can also be set to be complementary sequences hybridizing with positions, which are partially common but are deviated from each other. Even in this case, complete match, mismatch or bulge formation, the designing of a DNA/RNA chimera, etc. may be selected, as appropriate, for the first sequence and the second sequence.

When the RNA oligomer comprises "a second sequence that is different from the first sequence," the first sequence and the second sequence preferably have the same target sequence on the mRNA. The phrase "to have the same target sequence on the mRNA" means that the sequence on the mRNA with which the second sequence can hybridize is the same as that of the first sequence. Such a first sequence and a second sequence can be prepared, as appropriate, according to a known method or a method equivalent thereto. In some aspects, the second sequence has an identity of 90% or more (for example, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more) to the first sequence. In general, the numerical value of the identity is preferable, as the numerical value increases. Besides, the identity of RNA sequences can be determined using analysis software such as BLAST (see, for example, Altzshul S.F.et al., J. Mol. Biol. 215, 403 (1990)). In the case of using BLAST, the default parameters of each program are used.

In the case of the first sequence and the second sequence of the RNA oligomer that crosslink between two types of mRNAs, the first sequence is set to be a sequence hybridizing only with one type of mRNA, whereas the second sequence is set to be a sequence hybridizing only with the other type of mRNA, so that the two types of mRNAs can be crosslinked to each other by the RNA oligomers.

The range of "90% or more" in the phrase "to have an identity of 90% or more" regarding the RNA sequence is, for example, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more. In general, the numerical value of the above-described identity is preferable, as the numerical value thereof increases. Besides, the identity of RNA sequences can be determined using analysis software such as BLAST (see, for example, Altzshul S.F.et al., J. Mol. Biol. 215, 403 (1990)). In the case of using BLAST, the default parameters of each program are used.

The phrase "to hybridize with the mRNA" means that the RNA oligomer hybridizes with the mRNA under the after-mentioned hybridization conditions.

The first sequence and the second sequence of the RNA oligomer are designed to hybridize with the sequence of the mRNA consisting of consecutive 12 to 40 nucleotides (12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides).

The length of each of the first sequence and the second sequence of the RNA oligomer is preferably 12 to 30 nucleotides, more preferably 15 to 23 nucleotides, and further preferably 17 nucleotides. In some aspects, in addition to the aforementioned length of each of the first sequence and the second sequence of the RNA oligomer, the length of the linker sequence is preferably 0 to 100 nucleotides (0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 nucleotides), more preferably 0 to 30 nucleotides, and further preferably 10 nucleotides. In some aspects, the entire length of the RNA oligomer is, for example, 24 to 180 nucleotides, preferably 30 to 150 nucleotides, more preferably 40 to 100 nucleotides, further preferably 40 to 70 nucleotides, and particularly preferably 44 nucleotides.

Herein, the length of the linker sequence that is "0 nucleotide" means that the linker sequence is not present.

Preferably, the first sequence and the second sequence of the RNA oligomer each consist of a sequence of 12 to 30 nucleotides complementary to the sequence of the mRNA. More preferably, the sequence of the RNA oligomer consists of a sequence of 15 to 23 nucleotides complementary to the sequence of the mRNA. Further preferably, the sequence of the RNA oligomer consists of a sequence of 17 nucleotides complementary to the sequence of the mRNA.

The linker sequence is not particularly limited, as long as it is a sequence having a chain length consisting of 0 to 100 nucleotides. In some aspects, the linker sequence is designed not to hybridize with or to hardly hybridize with the sequence on the mRNA. The linker sequence preferably consists of a sequence that does not form a complementary strand to the sequence on the mRNA. Since the adenine (A) : uracil (U) bond is weaker than the guanine (G) : cytosine (C) bond, A and U are preferably used as nucleotides for the linker sequence. In particular, since U is likely to hybridize with a poly A chain on the mRNA, A is more preferably used. In some preferred aspects, the linker sequence is a sequence consisting of nucleotides that are all adenines.

The position in the mRNA, with which the RNA oligomer is allowed to hybridize, is any position of 5' UTR, a coding region, 3' UTR, and a poly A sequence. The RNA oligomer is desirably designed, such that it predicts the secondary structure of the mRNA, and hybridizes with a portion of the mRNA strand that does not have the secondary structure. That is to say, the RNA oligomer is preferably designed to hybridize with a portion not having the secondary structure in the entire mRNA sequence. The software that predicts the secondary structure of the mRNA is, for example, the one described in the Examples later.

In some aspects, the RNA oligomer is designed to hybridize with the coding region of the mRNA.

As RNA oligomers used in the production of the complex, for example, at least two types of RNA oligomers are used with respect to one type of mRNA. The term "one type of mRNA" means a single mRNA group comprising the same sequences. One type of RNA oligomer means a single RNA oligomer group comprising the same sequences as first sequences. The term "at least two types of RNA oligomers" means that there are two or more of such RNA oligomer groups and individual RNA oligomer groups each comprise different first sequences. Preferably 2 to 50 types of, more preferably 2 to 30 types of, and further preferably 2 to 8 types of RNA oligomers are used with respect to one type of mRNA in the production of a complex. As the number of the RNA oligomers increases, the stability of the mRNA is further improved. When the RNA oligomers used in the production of a complex are in the range of 2 to 50 types, the translation efficiency of the mRNA can be maintained at a relatively high level.

In some aspects, when at least two types of RNA oligomers are used, the RNA oligomers are designed such that individual RNA oligomers are not overlapped on the mRNA.

In some aspects, for example, at least one type of the RNA oligomer is used with respect to one type of the mRNA. In this case, the RNA oligomers used in the production of a complex are preferably of 1 to 50 types, more preferably of 1 to 30 types, and further preferably 1 to 8 types, with respect to one type of the mRNA. As the number of the RNA oligomers increases, the stability of the mRNA is further improved. When the RNA oligomers used in the production of a complex are in the range of 1 to 50 types, the translation efficiency of the mRNA can be maintained at a relatively high level.

In some aspects, one type of mRNA is comprised in one complex. In some other aspects, two or more types of (e.g., 2, 3, 4, 5, 6, 7, 8, 9 or 10 types of) mRNAs are comprised in one complex.

Preferably, with regard to one type of mRNA, the concentration of the mRNA used in the production of complex is, for example, 1 µg/mL to 10,000 µg/mL, more preferably 10 µg/mL to 10,000 µg/mL, and further preferably 50 µg/mL to 10,000 µg/mL. Examples of the solvent for the mRNA upon the generation of a complex may include a phosphate buffer, a normal saline, distilled water, a Tris buffer, and a HEPES buffer. Among these, a phosphate buffer or a normal saline is preferable, and a normal saline is more preferable. It is assumed that, by increasing the salt concentration, electrostatic interaction will be suppressed, hydrogen bonds will become dominant, and easy hybridization will be provided, and consequently, the formation of a complex will be promoted.

At this time, the molar ratio between one type of the mRNA and each RNA oligomer is, for example, 1 : 0.05 to 0.05 : 1, preferably 1 : 0.1 to 0.1 : 1, and more preferably 1 : 0.3 to 0.3 : 1. Herein, regarding the molar ratio between one type of the mRNA and each RNA oligomer, 1 : 0.5 is theoretically the most favorable value. The molar ratio 1 : 0.5 indicates a state in which the mRNAs and complementary sequences on linkers all appropriately bind to one another, assuming that hybridizations have been all achieved.

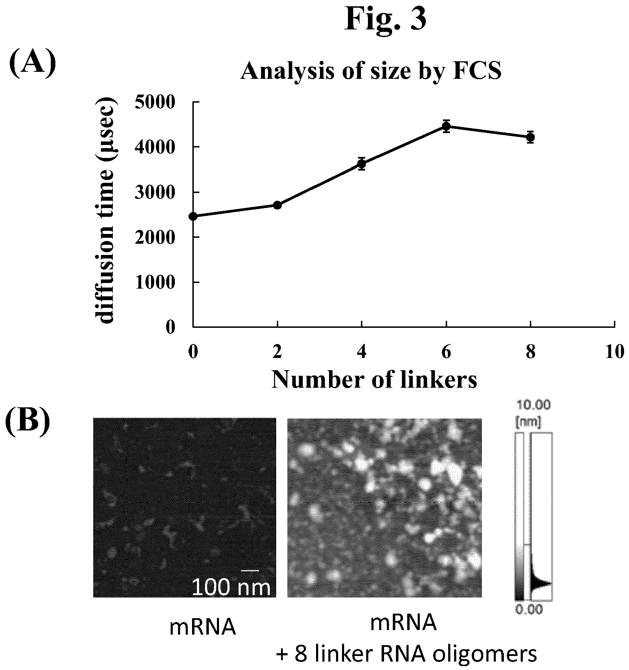

The complex is an aggregate in which a plurality of mRNAs and a plurality of RNA oligomers are gathered as a result of the crosslinking of the mRNAs by the RNA oligomers. The size of such a complex is preferably 10 nm to 10,000 nm, more preferably 30 nm to 1,000 nm, and further preferably 50 nm to 500 nm. Taking into consideration elements such as the concentration of the mRNA, the salt concentration, the number of the RNA oligomers, etc. upon preparation of a complex, the size of the complex can be changed, as appropriate. Specifically, as the mRNA concentration increases upon preparation of a complex, the formation of a large aggregate is expected. In addition, as the salt concentration increases, electrostatic interaction will be suppressed, hydrogen bonds will become dominant, and easy hybridization will be provided, and consequently, the formation of a large complex will be assumed. Moreover, by increasing the types of RNA oligomers used in preparation of a complex, the formation of a large aggregate is expected, as shown in Figure 3(A).

Production of a complex, namely, hybridization of the RNA oligomer(s) with the mRNA can be carried out by applying a known method and known conditions. In the hybridization, the reaction mixture is heated and is then left at rest for a certain period of time, and thereafter, the temperature is gradually decreased. The heating is carried out for the purpose of dissociating complementary bonds previously existing in the molecules of the mRNA or the oligomer(s), or among the molecules thereof, and thereby more efficiently binding the mRNA with the oligomer(s). The temperature and the time can be adjusted, as appropriate, as long as suitable hybridization can be guaranteed. Further, moderate temperature decrease brings on an increase in the hybridization specificity. In addition, the chain length of the oligomer does not largely influence on determination of hybridization conditions. Hybridization conditions should be determined, as appropriate, while evaluating hybridization efficiency. For example, the methods and conditions described in the Examples later are applied.

Moreover, the present invention provides a method for stabilizing an mRNA, comprising forming a complex consisting of an mRNA encoding a target gene and at least two types of RNA oligomers comprising the following RNA sequence (a) or (b), wherein
at least the two types of RNA oligomers are capable of hybridizing with each different sequence in the sequence of the mRNA, and
in the complex, a first sequence of the RNA oligomer hybridizes with an mRNA that is different from an mRNA for a second sequence of the RNA oligomer:
(a) an RNA sequence comprising a first sequence of 12 to 40 nucleotides complementary to the mRNA sequence, a linker sequence of 0 to 100 nucleotides, and a second sequence that is the same as the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence that is the same as the first sequence, in this order.

Furthermore, the present invention provides a method for stabilizing an mRNA, comprising forming a complex consisting of an mRNA encoding a target gene and at least one type of RNA oligomer comprising the following RNA sequence (a) or (b), wherein
in the complex, a first sequence of the RNA oligomer hybridizes with an mRNA that is different from an mRNA for a second sequence of the RNA oligomer:
(a) an RNA sequence comprising a first sequence of 12 to 40 nucleotides complementary to the mRNA sequence, a linker sequence of 0 to 100 nucleotides, and a second sequence that is a sequence of 12 to 40 nucleotides complementary to the mRNA sequence and is different from the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence having an identity of 90% or more to the sequence of 12 to 40 nucleotides complementary to the mRNA sequence, being capable of hybridizing with the mRNA, and being different from the first sequence, in this order.

The mRNA and the RNA oligomer(s) used in the stabilization methods are as described in the explanations regarding the above-described complex.

### 3. Carrier

In some aspects, the complex comprising the mRNA is in a naked form. That is, the complex comprising the mRNA is not loaded into carriers.

In some other aspects, the complex comprising the mRNA is in a form in which it is loaded in a carrier. The type of such a carrier is not particularly limited, as long as it can load the complex comprising the mRNA therein and can deliver it to a preferred site in the body of a subject. The carrier is a lipidic mRNA carrier or a cationic polymer complex, and more preferably a polymeric micelle or a lipidic mRNA carrier.

The polymeric micelle has a two-layer structure consisting of an inner core formed with a condensed nucleic acid and a cationic polymer, and an outer shell formed with a hydrophilic polymer. The cationic polymer is, for example, a polyamino acid derivative. The hydrophilic polymer is, for example, polyethylene glycol ("PEG"). The inner core physically or chemically encapsulates the mRNA therein. The outer shell determines the physicochemical properties of the polymeric micelle under the body's internal environment by its physicochemical properties. For example, in the case of PEG, it can suppress the non-specific adsorption of surrounding substances onto the polymeric micelle, and also can avoid the foreign matter recognition of the polymeric micelle in a living body. The polymeric micelle can enter the cells by endocytosis. The polymeric micelle can utilize, for example, the interaction of a polycation with a nucleic acid on a block polymer (polyion complex ("PIC")), and can also utilize a hybrid micelle of the polyion complex with an inorganic molecule. Examples of the PIC-type polymeric micelle may include PIC micelles formed by association of mRNA with PEG-PAsp (DET)-Chol, PEG-PAsp (DET), or PEG-PLys, or other polycations such as PAsp(TET) or PAsp(TEP) used in block copolymers (Uchida, S., et al., Biomaterials (2016) 82, pp. 221-228), and those used in triblock copolymers (Osawa, S., et al., Biomacromolecules 17, pp. 354-361 (2016)). Examples of the hybrid micelle with an inorganic molecule may include PEGylated calcium phosphate (CaP) particles (Pittela, et al., Biomaterials (2011) 32, pp. 3106-3114) and PEGylated silica particles (Miyata, K., et al., Biomaterials (2010) 31, pp. 4764-4770).

The lipidic mRNA carrier is formed using a lipid or a cationic lipid as a carrier, and an mRNA is loaded in or bound to the carrier. The lipidic mRNA carrier is obtained by mixing an mRNA with one or more selected from the group consisting of: for example, cationic lipids such as N-[1-(2,3-dioleyloxy) propyl]-N,N,N-trimethylammonium chloride (DOTMA), 2,3-dioleyloxy-N-[2-(spermine carboxamido)ethyl]-N, N-dimethyl-1-propanaminium trifluoroacetic acid (DOSPA), 1,2-dioleoyl-3-(trimethylammonium)propane (DOTAP), N-[1 -(2,3 -dimyristyloxy)propyl]-N,N-dimethyl-N-(2-hydroxyethyl) ammonium bromide (DMRIE), or DC-Cholesterol; neutral phospholipids such as distearoylphosphatidylcholine (DSPC) or dioleoyl phosphatidylethanolamine (DOPE); PEGylated lipids; and cholesterols.

The cationic polymer complex is a mixture of an mRNA and, for example, linear or branched polyethylenimine, polylysine, polyarginine, a chitosan derivative, or a polymethacrylic acid derivative.

These carriers can be prepared by a known method or a method equivalent thereto.

In some aspects, the amount of a complex comprising an mRNA loaded in the carrier is, for example, 0.5 to 200, preferably 1 to 50, and more preferably 1 to 10, in terms of the ratio between the cationic charge (+) in the carrier and the anionic charge (-) of the complex comprising the mRNA (+/- ratio).

### 4. Pharmaceutical composition

The present invention provides a pharmaceutical composition comprising the above-described complex. In some aspects, the complex is loaded in a carrier. The pharmaceutical composition is used to deliver the above-described complex into the body of a subject.

The "subject" is a human, or an organism other than the human, such as, for exam ple, birds and non-human mammals (e.g., bovines, monkeys, cats, mice, rats, Guinea pigs, hamsters, pigs, dogs, rabbits, sheep and horses).

The pharmaceutical composition can be administered to the subject via intravenous administration, intraarterial administration, oral administration, intratissue administration (e.g., intravesical administration, intrathoracic administration, intraperitoneal administration, intraocular administration, and intracerebral administration), transdermal administration, transmucosal administration, transpulmonary administration, or transrectal administration. In particular, intravenous administration, transdermal administration, and transmucosal administration are desirable. Dosage forms suitable for these administrations are, for example, various injections, oral agents, infusions, inhalants, eye drops, ointments, lotions, and suppositories. Administration conditions, such as applied dose, the number of administrations and administration period, can be determined, as appropriate, by a person skilled in the art, while taking into consideration the type of the mRNA, dosage form, the conditions of the subject, such as age or bodyweight, administration route, and the properties or degree of the disease.

The pharmaceutical composition can be used in a treatment of introducing an mRNA encoding a desired gene into cells causing various types of diseases. Accordingly, the present invention can also provide a method for treating various types of diseases, comprising administering the pharmaceutical composition to a subject in need of the treatment of various types of diseases. It is to be noted that various conditions such as applied dose, the number of administrations and administration period are the same as those described above.

Examples of various diseases to be treated with the pharmaceutical composition may include cancers, viral diseases, metabolic diseases, circulatory diseases, neurological diseases, renal urinary diseases, hematologic malignancies, diseases desiring promotion or suppression of apoptosis, collagen diseases, respiratory diseases, and gastrointestinal diseases.

For the pharmaceutical composition, agents that are commonly used in drug manufacturing, such as excipients, fillers, thickeners, binders, wetting agents, disintegrators, lubricants, surfactants, dispersants, buffers, preservatives, solubilizing agents, antiseptics, flavoring agents, soothing agents, stabilizers, and isotonic agents, may be appropriately selected and used, and the pharmaceutical composition can be prepared according to a conventional method, using these agents. When an intravenous injection (including infusion) is produced for example, it is provided in the state of a unit dose ampule, a multidose container, or the like.

In some aspects, while taking into consideration the type of the mRNA, dosage form, the conditions of the subject, such as age or bodyweight, administration route, and the properties or degree of the disease, in general, the applied dose of the mRNA is, for example, in the range of 0.1 mg to 5 g/human adult per day, and preferably in the range of 1 mg to 2 g/human adult per day. The applied dose may be different depending on the type of a target disease, administration form, and a target molecule in some cases. Accordingly, the applied dose is sufficient, even if it is less than the aforementioned dose in some cases. In contrary, the dose that is greater than the aforementioned range is necessary in some other cases. In addition, the pharmaceutical composition can be administered once or divided over several administrations per day, or with intervals of one to several days.

### 5. Kit for mRNA delivery

The kit for mRNA delivery of the present invention is characterized in that it includes the above-described complex. In some aspects, the complex is loaded in a carrier. The present kit can be preferably used, for example, in a method for treating various diseases, in which the above-described complex is used.

In the kit, the storage state of the complex is not limited. Taking into consideration stability (storability), the ease of use, etc., the state of a solution, powder or the like can be selected.

The kit may include other components, as well as the above-described complex. Examples of such other components may include various types of buffers and an instruction manual (use manual).

The present invention will be described in more detail in the following Examples. However, it should not be understood that the present invention is limited to these Examples.

### Examples

### Example 1: Preparation of mRNA

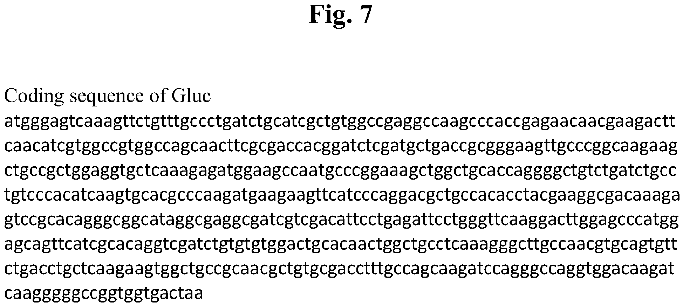

An mRNA is generally prepared from a template DNA according to *in vitro* transcription. Herein, a template DNA was produced as follows. First, a T7-Gluc plasmid was produced by inserting a Gluc coding sequence (Figure 7 and SEQ ID NO: 1) from pCMV-Gluc control plasmid (New England BioLabs, Ipswich, MA, USA) into the HindIII-Xbal site of a pSP73 vector (Promega). Thereafter, a T7-Gluc poly A120 plasmid was produced by inserting A120-(BsmBI cleavage site) into theEcoR1-Bgl2 site of the T7-Gluc plasmid. Thereafter, the resultant was cleaved with BsmBI, and was then blunt-ended with T4 DNA Polymerase (Takara Bio, Inc.), and the obtained product was then used in the subsequent *in vitro* transcription.

Using mMESSAGE mMACHINE (trade name) T7 (Thermo Fisher Scientific), the above-obtained product encoding Gaussia luciferase (Gluc) was subjected to a restriction enzyme treatment, and the blunt-ended template DNA (T7-Gluc poly A120 plasmid) was then transcribed under *in vitro* environment, so as to produce an mRNA. The transcribed mRNA was purified using RNeasy Mini Kit (QIAGEN), and was then recovered. The concentration of the recovered mRNA was measured using NanoDrop (Thermo Fisher Scientific). Moreover, whether the target mRNA was produced was confirmed by electrophoresis using Agilent 2100 Bioanalyzer (Agilent technologies).

Using NanoDrop, it was confirmed that a high-purity mRNA, in which the ratio between the absorbance at 260 nm and the absorbance at 280 nm was 2.0 to 2.2, was produced at a high concentration of 500 to 1,000 ng/µL. Moreover, according to the analysis using the Bioanalyzer, production of an mRNA having the intended size was confirmed.

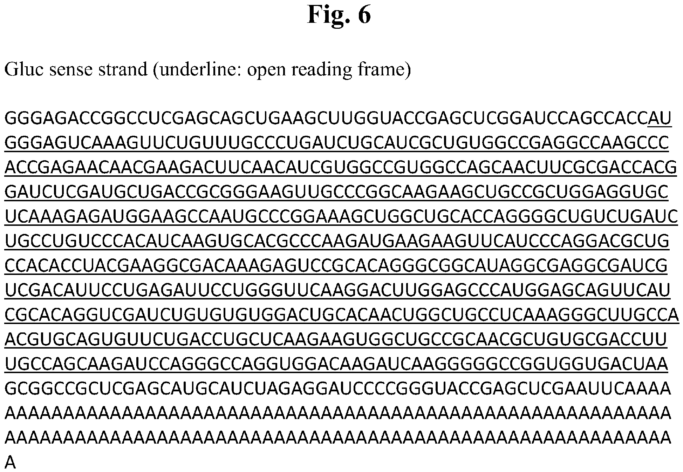

The sequence of the produced mRNA (SEQ ID NO: 2) is shown in Figure 6. In the sequence shown in Figure 6, the underlined portion indicates an open reading frame (ORF). 5' UTR (54 nucleotides) is located upstream of the ORF and 3' UTR (52 nucleotides) is located downstream of the ORF. 120A located further downstream thereof indicates a poly A sequence.

Herein, regarding the number of Poly A, theoretically, 120 bp are incorporated into the template DNA, and in mRNA, it is 119 nucleotides. However, the number of Poly A may be increased or decreased at the stage of DNA amplification or preparation of the mRNA.

### Example 2: Hybridization of RNA oligomer

An RNA oligomer was designed and prepared, as follows. Using RNA secondary structure predicting software (http://rtips.dna.bio.keio.ac.jp/ipknot/), the secondary structure of the Gluc mRNA was predicted, and an RNA oligomer was then designed with respect to a portion of the RNA strand, which did not have a secondary structure. The present inventors asked Hokkaido System Science Co., Ltd. to synthesize the RNA oligomer, including cholesterol modification on the 5'-terminus or 3'-terminus. It is to be noted that, in the following RNA oligomers, the "linker RNA oligomer" means an RNA oligomer comprising a 17-mer sequence capable of hybridizing with the mRNA, a 10-mer linker sequence whose nucleotides are all adenines, and a sequence that is the same as the 17-mer sequence, in this order. On the other hand, the "non-linker RNA oligomer" has a structure that may be a half of the aforementioned linker RNA oligomer, and it is an RNA oligomer comprising a 17-mer sequence capable of hybridizing with the mRNA and a 5-mer linker sequence whose nucleotides are all adenines, in this order.

### RNA oligomers

### Linker RNA oligomers

Linker RNA oligomer 1: UCUUUGAGCACCUCCAGAAAAAAAAAAUCUUUGAGCACCUCCAG (SEQ ID NO: 3)
Linker RNA oligomer 2: GCGGCAGCCACUUCUUGAAAAAAAAAAGCGGCAGCCACUUCUUG (SEQ ID NO: 4)
Linker RNA oligomer 3: ACUCUUUGUCGCCUUCGAAAAAAAAAAACUCUUUGUCGCCUUCG (SEQ ID NO: 5)
Linker RNA oligomer 4: CUCGGCCACAGCGAUGCAAAAAAAAAACUCGGCCACAGCGAUGC (SEQ ID NO: 6)
Linker RNA oligomer 5: GUGGGACAGGCAGAUCAAAAAAAAAAAGUGGGACAGGCAGAUCA (SEQ ID NO: 7)
Linker RNA oligomer 6: GCAGCCAGCUUUCCGGGAAAAAAAAAAGCAGCCAGCUUUCCGGG (SEQ ID NO: 8)
Linker RNA oligomer 7: UUGAAGUCUUCGUUGUUAAAAAAAAAAUUGAAGUCUUCGUUGUU (SEQ ID NO: 9)
Linker RNA oligomer 8: CUCUAGAUGCAUGCUCGAAAAAAAAAACUCUAGAUGCAUGCUCG (SEQ ID NO: 10)

### Non-linker RNA oligomers

Non-linker RNA oligomer 1: UCUUUGAGCACCUCCAGAAAAA (SEQ ID NO: 11)
Non-linker RNA oligomer 2: GCGGCAGCCACUUCUUGAAAAA (SEQ ID NO: 12)
Non-linker RNA oligomer 3: ACUCUUUGUCGCCUUCGAAAAA (SEQ ID NO: 13)
Non-linker RNA oligomer 4: CUCGGCCACAGCGAUGCAAAAA (SEQ ID NO: 14)
Non-linker RNA oligomer 5: GUGGGACAGGCAGAUCAAAAAA (SEQ ID NO: 15)
Non-linker RNA oligomer 6: GCAGCCAGCUUUCCGGGAAAAA (SEQ ID NO: 16)
Non-linker RNA oligomer 7: UUGAAGUCUUCGUUGUUAAAAA (SEQ ID NO: 17)
Non-linker RNA oligomer 8: CUCUAGAUGCAUGCUCGAAAAA (SEQ ID NO: 18)

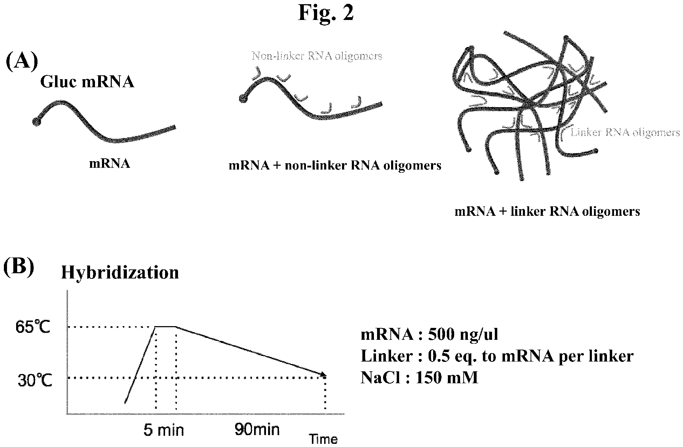

Each RNA oligomer was added to a 150 mM NaCl solution (Wako Pure Chemical Industries, Ltd.) containing 500 ng/µL mRNA, in an amount of 0.5 equivalents with respect to the amount of the mRNA (molar ratio), and the obtained mixture was heated at 65°C for 5 minutes and was then cooled to 30°C over 90 minutes, so as to perform hybridization (Figure 2(B)). Thereby, a complex of the mRNA and the RNA oligomers was produced ("mRNA + linker RNA oligomers" of Figure 2(A)). Likewise, "mRNA + non-linker RNA oligomers" was also produced. However, in the case of the "mRNA + non-linker RNA oligomers," the RNA oligomer was added in an amount of 1 equivalent with respect to the mRNA (molar ratio) (Figure 2(A)). This means that the "mRNA + non-linker RNA oligomers" is a control in which the RNA oligomers are formed but the mRNAs are not crosslinked to one another and thus, a nano-assembly is not formed. Besides, in Figure 2(A), a single-stranded mRNA is also shown.

Hereafter, a complex of an mRNA and an RNA oligomer(s) that is generated as a result of the hybridization is referred to as an "mRNA nano-assembly."

### Example 3: Analysis of physical properties of mRNA nano-assembly

### (1) Analysis by FCS (Fluorescence Correlation Spectroscopy, manufactured by OLYMPUS, MF20)

The size of an mRNA nano-assembly was evaluated by FCS analysis, and the optimal number of linkers was studied. The method of producing the mRNA nano-assembly is as described in Example 2.

### Types of used RNA oligomers

In experiments in which the number of linkers (i.e., the types of the used RNA oligomers) was 2, 4, 6, or 8, the following RNA oligomers, each of which was described in Example 2, were used.
2 linkers: linker RNA oligomers 3 and 5
4 linkers: linker RNA oligomers 2, 4, 6 and 8
6 linkers: linker RNA oligomers 1, 2, 4, 5, 6 and 8
8 linkers: linker RNA oligomers 1, 2, 3, 4, 5, 6, 7 and 8

An mRNA was stained with the fluorescent dye Cy5, and an mRNA nano-assembly was then formed. Thereafter, the mRNA nano-assembly was then set to be 5 ng/µL (in distilled water), and the measurement was then carried out. For Staining of the mRNA with the fluorescent dye, Label IT tracker Cy5 kit (Mirus) was employed.

The results are shown in Figure 3(A). As the numerical value shown in the longitudinal axis in Figure 3(A) (diffusion time (µsec)) increases, it means that diffusion was slow and the particle diameter of the mRNA nano-assembly became large. When the number of linkers was 6 or greater, the size of the mRNA nano-assembly became constant. Hence, in the subsequent examples, regarding the number of likers, 8 linkers were mainly selected for leaving some margin.

### (2) Analysis by AFM (Atomic Force Microscopy, Shimadzu Corporation, SPM-9700)

The concentration of the mRNA was set to be 3 ng/µL (in 10 mM MgCl₂ solution, (Wako Pure Chemical Industries, Ltd.)), and thereafter, it was added onto a substrate and was then dried. Subsequently, the measurement was carried out by AFM.

The results are shown in Figure 3(B). A color scale is shown in the right side of Figure 3(B). This color scale indicates that, as the color is close to white, the height increases, namely, the size increases. An mRNA nano-assembly comprising 8 linkers includes many white portions, and there are observed those having a particle diameter of larger than 100 nm. On the other hand, in the case of a single mRNA, it includes a few white portions, and almost no portions have a particle diameter of larger than 100 nm. Thus, it is found that the mRNA nano-assembly has a larger particle diameter than the single mRNA does.

### Example 4: Enzyme resistance of mRNA nano-assembly and translation activity in cell-free translation system

### (1) Enzyme resistance test

The enzyme resistance of an mRNA nano-assembly in fetal bovine serum (FBS, Dainippon Sumitomo Pharma Co., Ltd., Osaka, Japan) was examined.

Fetal bovine serum (FBS) was diluted with a phosphate buffer (PBS, Wako Pure Chemical Industries, Ltd.) to a predetermined concentration, and an mRNA nano-assembly solution was then added to the diluted solution, so that the final concentration of the mRNA became 6.25 ng/µL. The mixed solution was left at rest at 37°C for 15 minutes. Thereafter, the mRNA was extracted from the resulting solution, using RNeasy Mini Kit (QIAGEN). The extracted mRNA was reverse-transcribed to complementary DNA (cDNA), and the amount of remaining cDNA was quantified and evaluated according to qRT-PCR. The result was shown as a relative value, while the amount of mRNA that had not been left at rest in the presence of FBS at 37°C was set at 100%.

The following primers were used in the qRT-PCR measurement.
Forward target sequence: TGAGATTCCTGGGTTCAAGG (SEQ ID NO: 19)
Reverse target sequence: GTCAGAACACTGCACGTTGG (SEQ ID NO: 20)

The results are shown in Figure 4(A). The "mRNA + 8 non-linker RNA oligomers" means an mRNA to which 8 non-linker RNA oligomers (non-linker RNA oligomers 1 to 8) were added as RNA oligomers. The enzyme stability of the "mRNA + 8 non-linker RNA oligomers" was slightly improved in comparison to the mRNA. This is considered because a double strand was formed between the mRNA and the RNA oligomer. The "mRNA + 8 linker RNA oligomers" means an mRNA to which 8 linker RNA oligomers (linker RNA oligomers 1 to 8) were added as RNA oligomers. The enzyme stability of the "mRNA + 8 linker RNA oligomers" became 100 times higher than that of the "mRNA + 8 non-linker RNA oligomers." This can be calculated based on the results that the FBS concentration in which 50% mRNA is decomposed is approximately 0.005% in the case of the "mRNA + 8 non-linker RNA oligomers," whereas it is approximately 1% in the case of "mRNA + 8 linker RNA oligomers." From the aforementioned results, it was found that the enzyme resistance of the mRNA is significantly improved by forming an mRNA nano-assembly.

It is to be noted that, as statistical analyses, a Tukey's test was carried out after completion of an ANOVA (analysis of variance) test. The symbol *** indicates that the mRNA + 8 linker RNA oligomers group is p < 0.001, with respect to both the single mRNA group and the mRNA + 8 non-linker RNA oligomer group.

### (2) Translation activity in cell-free translation system

The mRNA nano-assembly was added to Rabbit reticulocyte lysate (Promega), and the obtained mixture was then incubated at 37°C for 4 hours. Thereafter, the luminescence intensity of Gluc was measured. The luminescence intensity was measured using Renilla luciferase assay kit (Promega). As a luminometer, Mithras LB 940 manufactured by Berthold technologies was used.

The results are shown in Figure 4(B). In the figure, "+ 8 linkers" means an mRNA to which 8 linker RNA oligomers (linker RNA oligos 1 to 8) were added as RNA oligomers. The translation activity upon addition to the cell-free translation system was hardly changed between the single mRNA and the "+ 8 linkers."

It is to be noted that a Student's t-test was carried out for statistical analysis, and that n. s. indicates no significance.

From the aforementioned results, it is found that the mRNA nano-assembly can improve the enzyme resistance of the mRNA, while maintaining the translation activity thereof.

### Example 5: Intracellular uptake of mRNA nano-assembly, and expression of mRNA in cells

HuH-7 cells (Riken Cell Bank) were seeded at a density of 10,000 cells/well on a 96-well plate. Twenty-four hours later, the medium was replaced with the serum-free medium Opti-MEM (Thermo Fisher Scientific), and 250 ng of mRNA was then added thereto. Four hours after addition of the mRNA, the measurement was carried out (Figure 5(A)).

### (1) Intracellular uptake of mRNA nano-assembly

With regard to the intracellular uptake amount of an mRNA, the mRNA was fluorescently labeled with Label IT tracker Cy3 kit (Mirus). Four hours after the introduction of the mRNA into the cells, the surface of the cells was washed with PBS twice, and the cells were then lysed with a Lysis buffer (Promega). Thereafter, using Infinite M1000 PRO spectrophotometer (Tecan Group Ltd., Mannedorf, Switzerland), the fluorescent intensity was observed at an excitation wavelength of 543 nm and a detection wavelength of 568 nm.

The results are shown in Figure 5(B). The "+ 8 linkers" means an mRNA to which 8 linker RNA oligomers (linker RNA oligomers 1 to 8) were added as RNA oligomers. The intracellular uptake amount of the mRNA was hardly changed between the single mRNA and the "+ 8 linkers."

It is to be noted that a Student's t-test was carried out for statistical analysis, and that n. s. indicates no significance.

### (2) Expression of mRNA in cells

With regard to the expression level of the mRNA in the cells, the medium was recovered 4 hours after the introduction of the mRNA, and the amount of Gluc contained in the recovered medium was then measured using Renilla luciferase assay kit (Promega).

The results are shown in Figure 5(C). The "+ 8 linkers" means an mRNA to which 8 linker RNA oligomers (linker RNA oligos 1 to 8) were added as RNA oligomers. The efficiency of the mRNA expression in the cells was hardly changed between the single mRNA and the "+ 8 linkers."

It is to be noted that a Student's t-test was carried out for statistical analysis, and that n. s. indicates no significance.

From the aforementioned results, it was found that the efficiency of the expression of the mRNA nano-assembly was not changed from that of the single mRNA, while the intracellular uptake efficiency of the mRNA nano-assembly was not changed from that of the single mRNA. These results suggest that the translation activity of the mRNA nano-assembly be at the same level as the translation activity of the single mRNA even in the cells.

### Example 6: Preparation of polymeric micelle (PM) consisting of mRNA nano-assembly and block copolymer

### (1) Synthesis of polymer

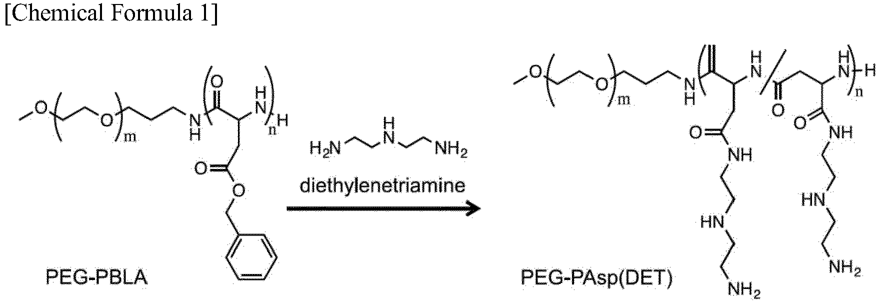

First, using α-methoxy-ω-amino-polyethylene glycol (MeO-PEG-NH₂, M_{w} of PEG: 12kDa) as an initiator, β-benzyl-L-aspartate-N-carboxylic acid anhydride (NCA-BLA) was subjected to ring-opening polymerization, so as to synthesize PEG-poly(β-benzyl L-aspartate) (PEG-PBLA). 505 mg of MeO-PEG-NH₂ was dissolved in benzene, and was then freeze-dried overnight. After completion of the freeze-drying, MeO-PEG-NH₂ and NCA-BLA were completely dissolved in a mixed solvent of dry dimethylformamide (DMF) : dry dichloromethane (DCM) (= 1 : 10) under Ar atmosphere. The NCA-BLA solution was added to the MeO-PEG-NH₂ solution, and the mixed solution was then stirred at 25°C for 3 days. Thereafter, the reaction solution was re-precipitated in an n-hexane/ethyl acetate (3 : 2) mixed solution to recover PEG-PBLA. Thereafter, PEG-PBLA was vacuum-dried to obtain white powders. The polymerization degree of PBLA was 68 (n = 68). Besides, in the above formula, m = 293.

PEG-PBLA (200 mg) was dissolved in benzene and was then freeze-dried, and the thus dried PEG-PBLA and dry diethylenetriamine (DET) (in an amount of 50 equivalents with respect to the amount of PBLA) were then dissolved in dry NMP containing 0.5 M thiourea. The thus obtained solution was cooled to 10°C, and the PEG-PBLA solution was then slowly added dropwise onto the DET solution, and the mixed solution was then stirred for 1 hour. After completion of the reaction, while the temperature of the solution was kept at 10°C or lower, the reaction solution was neutralized with a 5 N HCl aqueous solution, and was then dialyzed against a 0.01 N HCl aqueous solution at 4°C for 1 day. Thereafter, the resultant was dialyzed against ion exchange water at 4°C for one more day. After completion of the dialysis, the resulting solution was freeze-dried to obtain PEG-PAsp(DET) in the form of white powders. Besides, in the above formula, n = 68, and m = 293.

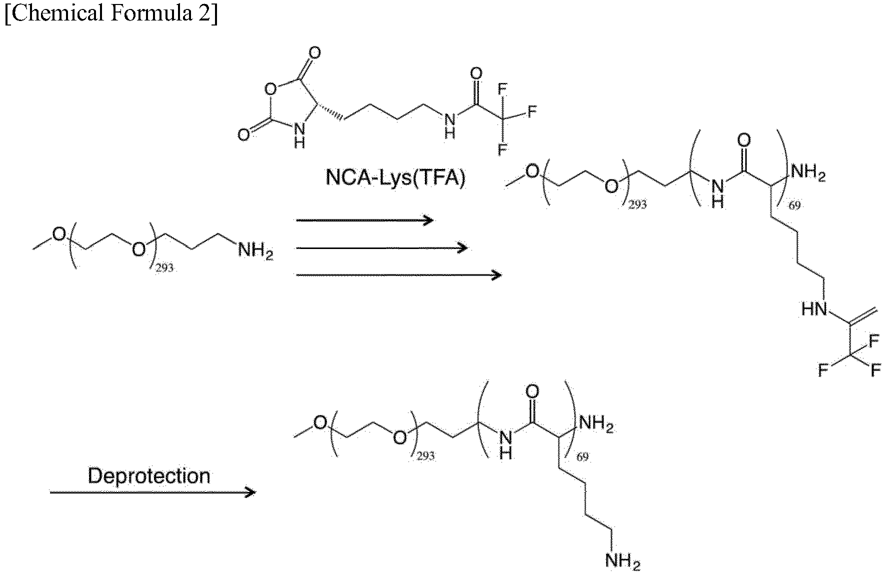

400 mg of MeO-PEG-NH₂ was dissolved in benzene, and was then freeze-dried overnight. After completion of the freeze-drying, MeO-PEG-NH₂ and NCA-Lys (TFA) were completely dissolved in a dry dimethylformamide (DMF) solvent containing 0.5 M thiourea under Ar atmosphere. The NCA-Lys (TFA) solution was added to the MeO-PEG-NH₂ solution, and the mixed solution was then stirred at 25°C for 3 days. Thereafter, the reaction solution was re-precipitated in diethyl ether, so as to recover PEG-PLys (TFA). After that, the recovered PEG-PLys (TFA) was vacuum-dried to obtain white powders. Thereafter, the resultant was allowed to react in a methanol mixed solvent containing 0.1 N NaOH at 35°C for 6 hours, so as to deprotect the TFA groups. The resultant was dialyzed against a 0.01 N HCl aqueous solution at 4°C for 1 day. After that, the resultant was dialyzed against ion exchange water at 4°C for one more day to obtain white powders of PEG-PLys. According to 1H-NMR analysis, the polymerization degree of PLys in the recovered PEG-PLys was found to be 69.

### (2) Preparation of micelles and measurement of particle diameter

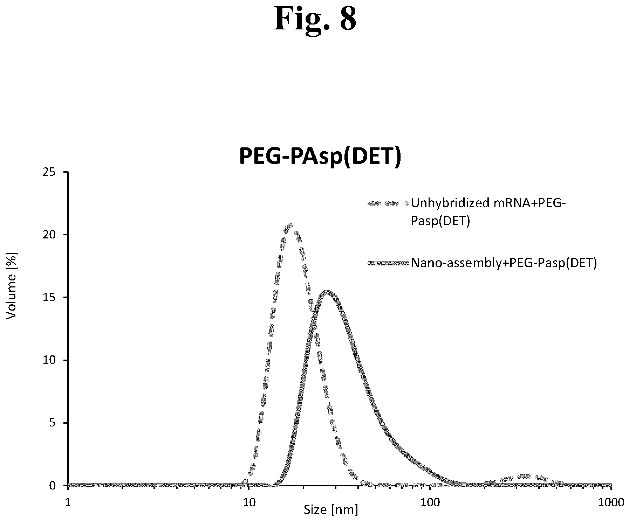

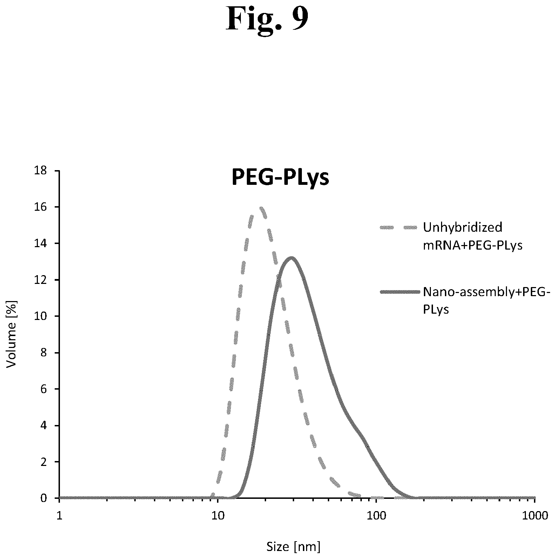

PEG-PAsp(DET) and a nano-assembly were each independently dissolved in a 10 mM HEPES buffer (pH 7.4). The concentration of the PEG-PAsp(DET) was adjusted, so that the ((positive charge number of PAsp(DET))/(negative charge number of mRNA nano-assembly)) (N⁺/P⁻ ratio) became 1.5 at pH 7.4. The PEG-PAsp(DET) solution was added to the mRNA nano-assembly solution, so that the final concentration of the mRNA became 33.3 µg/mL, thereby preparing polymeric micelles. In the case of PEG-PLys, the concentration of the PEG-PLys was adjusted, so that the ((positive charge number of PLys)/(negative charge number of mRNA nano-assembly)) (N⁺/P⁻ ratio) became 2 at pH 7.4, and PMs were then prepared in the same manner as in the case of the PEG-PAsp(DET). The particle diameter of the PMs was evaluated using ZetasizerNano (Malvern Instruments). The results are shown in Figure 8 and Figure 9. Even in the case of an mRNA that did not form an assembly (unhybridized mRNA), PMs were formed in the same manner as that described above.

Even in a case where a block copolymer was formed with the nano-assembly, particles with a particle diameter of 100 nm or less were formed. This is a size that is efficiently incorporated into various cells when the particles are administered into a living body.

### (2) Stability in serum

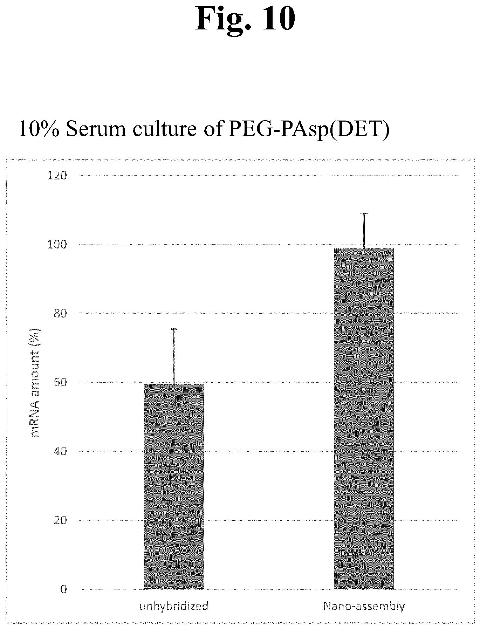

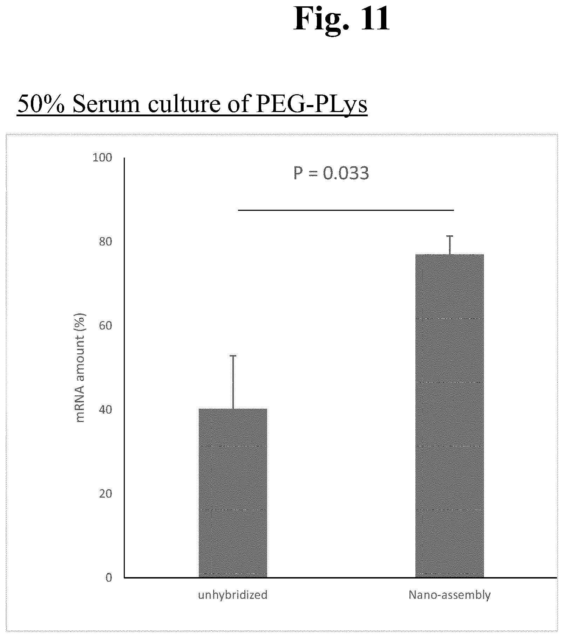

Fetal bovine serum (FBS) was diluted with a 10 mM HEPES buffer (pH 7.4) to a predetermined concentration, and a PM solution was then added to the diluted solution. The mixed solution was left at rest at 37°C for 15 minutes. Thereafter, the RNA was extracted from the resulting solution, using RNeasy Mini Kit (QIAGEN). The extracted mRNA was reverse-transcribed to complementary DNA (cDNA), and the amount of remaining cDNA was quantified and evaluated according to qRT-PCR. The result was shown as a relative value, while the amount of mRNA that had not been left at rest in the presence of FBS at 37°C was set at 100%. The subsequent experiment was carried out with N = 4 (Figures 10 and 11).

PMs consisting of nano-assemblies exhibited high enzyme resistance, in comparison to PMs consisting of mRNAs that did not form assemblies.

It is to be noted that a Student's t-test was carried out for statistical analysis, and that n. s. indicates no significance.

### Example 7: Preparation of nano-assembly from GFP mRNA

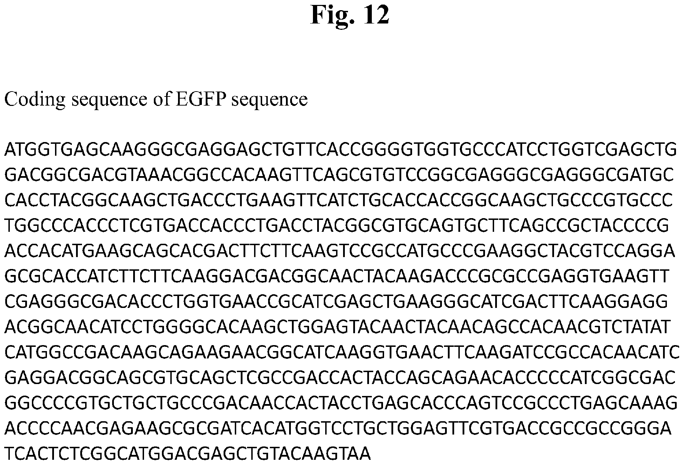

In the above-mentioned Examples, the results of analyzing the nano-assembly prepared from Gluc mRNA were shown. In order to demonstrate that the same strategy as that described above is effective even for other mRNAs, a nano-assembly prepared from GFP mRNA was analyzed in the present example. Herein, the coding sequence (SEQ ID NO: 21) of GFP is shown in Figure 12.

GFP mRNA (manufactured by Trilink) was purchased, and the 8 linker RNA oligomers (#17 to #24) synthesized by Hokkaido System Science Co., Ltd. were allowed to hybridize with the GFP mRNA by the same method as that in the case of the aforementioned Gluc of Example 2, so as to prepare nano-assemblies. The GFP mRNA comprises 5' UTR in a site upstream of the open reading frame (ORF), and also comprises 3' UTR downstream of the ORF, and a poly A sequence further downstream thereof.

The "linker RNA oligomer" is an RNA oligomer comprising a 17-mer sequence capable of hybridizing with the mRNA, a 10-mer linker sequence whose nucleotides are all adenines (A), and a sequence that is the same as the 17-mer sequence, in this order.

### Linker RNA oligomers

#17: CACCCCGGUGAACAGCUAAAAAAAAAACACCCCGGUGAACAGCU (SEQ ID NO: 22)
#18: CGCUGAACUUGUGGCCGAAAAAAAAAACGCUGAACUUGUGGCCG (SEQ ID NO: 23)
#19: CUUGCCGGUGGUGCAGAAAAAAAAAAACUUGCCGGUGGUGCAGA (SEQ ID NO: 24)
#20: GUGGUCGGGGUAGCGGCAAAAAAAAAAGUGGUCGGGGUAGCGGC (SEQ ID NO: 25)
#21: GCGCGGGUCUUGUAGUUAAAAAAAAAAGCGCGGGUCUUGUAGUU (SEQ ID NO: 26)
#22: GCCAUGAUAUAGACGUUAAAAAAAAAAGCCAUGAUAUAGACGUU (SEQ ID NO: 27)
#23: CUCGAUGUUGUGGCGGAAAAAAAAAAACUCGAUGUUGUGGCGGA (SEQ ID NO: 28)
#24: CUUCUCGUUGGGGUCUUAAAAAAAAAACUUCUCGUUGGGGUCUU (SEQ ID NO: 29)

### Example 8: Analysis of mRNA nano-assembly

The mRNA nano-assembly prepared in Example 7 was analyzed. The same methods as those in the case of Gluc mRNA of the aforementioned Examples were applied herein.

### (1) Analysis by FCS (Fluorescence Correlation Spectroscopy, manufactured by OLYMPUS, MF20)

The size of the mRNA nano-assembly was evaluated according to FCS analysis by the same method as that of Example 3(1).

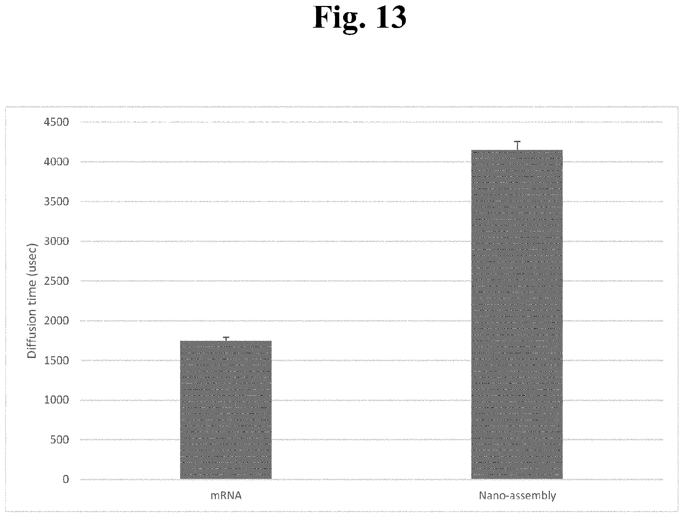

The results are shown in Figure 13. As the numerical value shown in the longitudinal axis in Figure 13 (diffusion time (µsec)) increases, it means that diffusion is slow and the particle diameter of the mRNA nano-assembly becomes large. It is found that the particle diameter of the GFP mRNA nano-assembly is almost the same as that of the Gluc mRNA nano-assembly (Figure 3(A)).

### (2) Enzyme resistance test

The enzyme resistance of the mRNA nano-assembly in fetal bovine serum (FBS, Dainippon Sumitomo Pharma Co., Ltd., Osaka, Japan) was examined by the same method as that of Example 4(1). However, the FBS concentration was fixed to be 1%.

Herein, the following primers were used in the qRT-PCR measurement.
Forward target sequence: AGAACGGCATCAAGGTGAAC (SEQ ID NO: 30)
Reverse target sequence: TGCTCAGGTAGTGGTTGTCG (SEQ ID NO: 31)

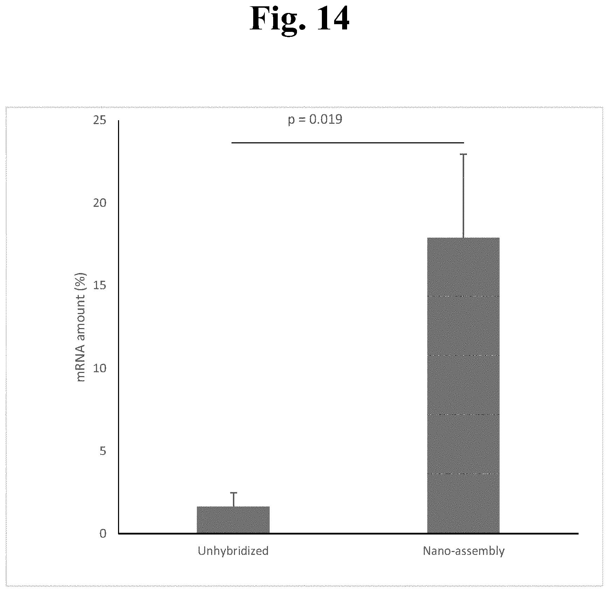

The results are shown in Figure 14. The "Nano-assembly" means an mRNA to which 8 linker RNA oligomers (#17 to #24) were added as RNA oligomers. In the "Nano-assembly," enzyme stability was more improved than in the mRNA ("Unhybridized").

It is to be noted that a Student's t-test was carried out for statistical analysis, and that n. s. indicates no significance.

### (3) Analysis by AFM (Atomic Force Microscopy, Shimadzu Corporation, SPM-9700)

The mRNA nano-assembly was measured according to AFM by the same method as that of Example 3(1).

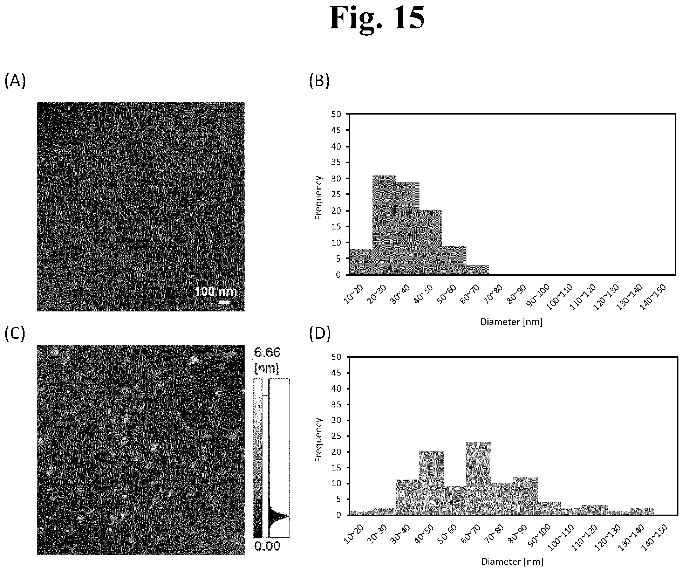

The results are shown in Figure 15(A) to (D). A color scale is shown in the right side of Figure 15(C). This color scale indicates that, as the color is close to white, the height increases, namely, the size increases. The mRNA nano-assembly comprising 8 linkers includes many white portions, and there are observed those having a particle diameter of larger than 100 nm (Figure 15(C) and (D)). On the other hand, in the case of a single mRNA, it includes a few white portions, and almost no portions have a particle diameter of larger than 100 nm (Figure 15(A) and (B)). From these matters, it is found that the mRNA nano-assembly has a larger particle diameter than the single mRNA does.

### Example 9: FFF (Field forward fractionation) analysis of mRNA nano-assembly

In FFF, separation can be carried out depending on the size of the mRNA nano-assembly, and large particles are separated as the time progresses.

Specifically, the GFP mRNA nano-assembly prepared in Example 7 was analyzed under the following conditions.
- Apparatus name: Eclipse AF4 (Shoko Scientific Co., Ltd.)
- mRNA concentration: 100 ng/µL
- Injected amount: 25 µL
- Flow rate: The detector flow was set at 1 mL/min, and the cross flow was reduced at a constant pace from 1 mL/min to 0 mL/min over 28 minutes.

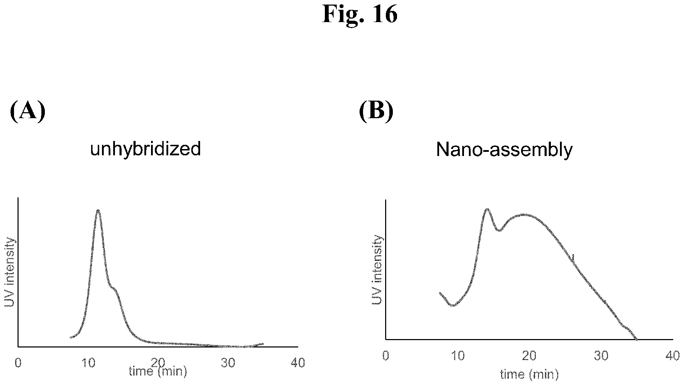

The results are shown in Figure 16. In Nano-assembly, the peak was shifted to the right, in comparison to unhybridize (Figure 16(B)), and the formation of large particles was confirmed (Figure 16(A)). In addition, according to this method, it is possible to separate nano-assemblies depending on the size thereof.

### Industrial Applicability

It is expected that mRNA delivery of using a complex consisting of an mRNA and RNA oligomers will be applied to the medical field as a method of supplying a therapeutic protein into a body safely and continuously.

### Sequence Listing Free Text

SEQ ID NO: 1 - Synthetic DNA
SEQ ID NOS: 2 to 18 - Synthetic RNA
SEQ ID NOS: 19 to 21 - Synthetic DNA
SEQ ID NOS: 22 to 29 - Synthetic RNA
SEQ ID NOS: 30 and 31 - Synthetic DNA

## Claims

1. A complex consisting of an mRNA encoding a target gene and RNA oligomers, wherein
the RNA oligomers are at least two types of RNA oligomers comprising the following RNA sequence (a) or (b),
at least the two types of RNA oligomers are capable of hybridizing with each different sequence in the sequence of the mRNA, and
a first sequence of the RNA oligomer hybridizes with an mRNA that is different from an mRNA for a second sequence of the RNA oligomer, so that the complex is formed:
(a) an RNA sequence comprising a first sequence of 12 to 40 nucleotides complementary to the mRNA sequence, a linker sequence of 0 to 100 nucleotides, and a second sequence that is the same as the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence that is the same as the first sequence, in this order.

2. The complex according to claim 1, wherein the RNA oligomers are at least two types of RNA oligomers comprising the following RNA sequence (a) or (b):
(a) an RNA sequence comprising a first sequence of 15 to 23 nucleotides complementary to the mRNA sequence, a linker sequence of 0 to 30 nucleotides, and a second sequence that is the same as the first sequence, in this order; or
(b) an RNA sequence comprising a first sequence having an identity of 90% or more to the sequence of 15 to 23 nucleotides complementary to the mRNA sequence and also being capable of hybridizing with the mRNA, a linker sequence of 0 to 30 nucleotides, and a second sequence that is the same as the first sequence, in this order.

3. The complex according to claim 1, wherein the RNA oligomers are at least two types of RNA oligomers comprising the following RNA sequence (a) or (b):
(a) an RNA sequence comprising a first sequence of 17 nucleotides complementary to the mRNA sequence, a linker sequence of 10 nucleotides, and a second sequence that is the same as the first sequence, in this order; or
(b) an RNA sequence comprising a first sequence having an identity of 90% or more to the sequence of 17 nucleotides complementary to the mRNA sequence and also being capable of hybridizing with the mRNA, a linker sequence of 10 nucleotides, and a second sequence that is the same as the first sequence, in this order.

4. The complex according to any one of claims 1 to 3, wherein the nucleotides of the linker sequence are all adenines.

5. The complex according to any one of claims 1 to 4, wherein at least the two types of RNA oligomers are 2 to 8 types of RNA oligomers.

6. The complex according to any one of claims 1 to 5, which comprises the mRNA and at least the two types of RNA oligomers, such that the concentration of the mRNA becomes 1 µg/mL to 10,000 µg/mL, and the molar ratio between the mRNA and each RNA oligomer of at least the two types of RNA oligomers becomes 1 : 0.05 to 0.05 : 1.

7. A pharmaceutical composition comprising the complex according to any one of claims 1 to 6.

8. A method for stabilizing an mRNA, comprising forming a complex consisting of an mRNA encoding a target gene and at least two types of RNA oligomers comprising the following RNA sequence (a) or (b), wherein
at least the two types of RNA oligomers are capable of hybridizing with each different sequence in the sequence of the mRNA, and
in the complex, a first sequence of the RNA oligomer hybridizes with an mRNA that is different from an mRNA for a second sequence of the RNA oligomer:
(a) an RNA sequence comprising a first sequence of 12 to 40 nucleotides complementary to the mRNA sequence, a linker sequence of 0 to 100 nucleotides, and a second sequence that is the same as the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence that is the same as the first sequence, in this order.

9. A complex consisting of an mRNA encoding a target gene and an RNA oligomer, wherein
the RNA oligomer is at least one type of RNA oligomer comprising the following RNA sequence (a) or (b), and
a first sequence of the RNA oligomer hybridizes with an mRNA that is different from an mRNA for a second sequence of the RNA oligomer, so that the complex is formed:
(a) an RNA sequence comprising a first sequence of 12 to 40 nucleotides complementary to the mRNA sequence, a linker sequence of 0 to 100 nucleotides, and a second sequence that is a sequence of 12 to 40 nucleotides complementary to the mRNA sequence and is different from the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence having an identity of 90% or more to the sequence of 12 to 40 nucleotides complementary to the mRNA sequence, being capable of hybridizing with the mRNA, and being different from the first sequence, in this order.

10. The complex according to claim 9, wherein the RNA oligomer is at least one type of RNA oligomer comprising the following RNA sequence (a) or (b):
(a) an RNA sequence comprising a first sequence of 15 to 23 nucleotides complementary to the mRNA sequence, a linker sequence of 0 to 30 nucleotides, and a second sequence that is a sequence of 15 to 23 nucleotides complementary to the mRNA sequence and is different from the first sequence, in this order; or
(b) an RNA sequence comprising a first sequence having an identity of 90% or more to the sequence of 15 to 23 nucleotides complementary to the mRNA sequence and also being capable of hybridizing with the mRNA, a linker sequence of 0 to 30 nucleotides, and a second sequence having an identity of 90% or more to the sequence of 15 to 23 nucleotides complementary to the mRNA sequence, being capable of hybridizing with the mRNA, and being different from the first sequence, in this order.

11. The complex according to claim 9, wherein the RNA oligomer is at least one type of RNA oligomer comprising the following RNA sequence (a) or (b):
(a) an RNA sequence comprising a first sequence of 17 nucleotides complementary to the mRNA sequence, a linker sequence of 10 nucleotides, and a second sequence that is a sequence of 17 nucleotides complementary to the mRNA sequence and is different from the first sequence, in this order; or
(b) an RNA sequence comprising a first sequence having an identity of 90% or more to the sequence of 17 nucleotides complementary to the mRNA sequence and also being capable of hybridizing with the mRNA, a linker sequence of 10 nucleotides, and a second sequence having an identity of 90% or more to the sequence of 17 nucleotides complementary to the mRNA sequence, being capable of hybridizing with the mRNA, and being different from the first sequence, in this order.

12. The complex according to any one of claims 9 to 11, wherein the nucleotides of the linker sequence are all adenines.

13. The complex according to any one of claims 9 to 12, wherein at least the one type of RNA oligomer is 2 to 8 types of RNA oligomers.

14. The complex according to any one of claims 11 to 13, which comprises the mRNA and at least the one type of RNA oligomer, such that the concentration of the mRNA becomes 1 µg/mL to 10,000 µg/mL, and the molar ratio between the mRNA and each RNA oligomer of at least the one type of RNA oligomer becomes 1 : 0.05 to 0.05 : 1.

15. A pharmaceutical composition comprising the complex according to any one of claims 11 to 14.

16. A method for stabilizing an mRNA, comprising forming a complex consisting of an mRNA encoding a target gene and at least one type of RNA oligomer comprising the following RNA sequence (a) or (b), wherein
in the complex, a first sequence of the RNA oligomer hybridizes with an mRNA that is different from an mRNA for a second sequence of the RNA oligomer:
(a) an RNA sequence comprising a first sequence of 12 to 40 nucleotides complementary to the mRNA sequence, a linker sequence of 0 to 100 nucleotides, and a second sequence that is a sequence of 12 to 40 nucleotides complementary to the mRNA sequence and is different from the first sequence, in this order; or
(b) an RNA sequence comprising a first sequence having an identity of 90% or more to the sequence of 12 to 40 nucleotides complementary to the mRNA sequence and also being capable of hybridizing with the mRNA, a linker sequence of 0 to 100 nucleotides, and a second sequence having an identity of 90% or more to the sequence of 12 to 40 nucleotides complementary to the mRNA sequence, being capable of hybridizing with the mRNA, and being different from the first sequence, in this order.

17. An mRNA carrier, which loads therein the complex according to any one of claims 1 to 6 and 11 to 14.

18. The mRNA carrier according to claim 17, wherein the carrier is a polymeric micelle or a lipidic mRNA carrier.

19. A pharmaceutical composition comprising the mRNA carrier according to claim 17 or 18.


SEQUENCE LISTING

<110>  THE UNIVERSITY OF TOKYO
       KAWASAKI INSTITUTE OF INDUSTRIAL PROMOTION

<120>  Method for stabilizing mRNA

<130>  G1922WO

<150>  US 62/583,691
<151>  2017-11-09

<160>  31

<170>  PatentIn version 3.5

<210>  1
<211>  558
<212>  DNA
<213>  Artificial sequence

<220>
<223>  synthetic DNA

<400>  1
atgggagtca aagttctgtt tgccctgatc tgcatcgctg tggccgaggc caagcccacc      60

gagaacaacg aagacttcaa catcgtggcc gtggccagca acttcgcgac cacggatctc     120

gatgctgacc gcgggaagtt gcccggcaag aagctgccgc tggaggtgct caaagagatg     180

gaagccaatg cccggaaagc tggctgcacc aggggctgtc tgatctgcct gtcccacatc     240

aagtgcacgc ccaagatgaa gaagttcatc ccaggacgct gccacaccta cgaaggcgac     300

aaagagtccg cacagggcgg cataggcgag gcgatcgtcg acattcctga gattcctggg     360

ttcaaggact tggagcccat ggagcagttc atcgcacagg tcgatctgtg tgtggactgc     420

acaactggct gcctcaaagg gcttgccaac gtgcagtgtt ctgacctgct caagaagtgg     480

ctgccgcaac gctgtgcgac ctttgccagc aagatccagg gccaggtgga caagatcaag     540

ggggccggtg gtgactaa                                                   558


<210>  2
<211>  783
<212>  RNA
<213>  Artificial sequence

<220>
<223>  synthetic RNA

<400>  2
gggagaccgg ccucgagcag cugaagcuug guaccgagcu cggauccagc caccauggga      60

gucaaaguuc uguuugcccu gaucugcauc gcuguggccg aggccaagcc caccgagaac     120

aacgaagacu ucaacaucgu ggccguggcc agcaacuucg cgaccacgga ucucgaugcu     180

gaccgcggga aguugcccgg caagaagcug ccgcuggagg ugcucaaaga gauggaagcc     240

aaugcccgga aagcuggcug caccaggggc ugucugaucu gccuguccca caucaagugc 300

acgcccaaga ugaagaaguu caucccagga cgcugccaca ccuacgaagg cgacaaagag 360

uccgcacagg gcggcauagg cgaggcgauc gucgacauuc cugagauucc uggguucaag 420

gacuuggagc ccauggagca guucaucgca caggucgauc ugugugugga cugcacaacu 480

ggcugccuca aagggcuugc caacgugcag uguucugacc ugcucaagaa guggcugccg 540

caacgcugug cgaccuuugc cagcaagauc cagggccagg uggacaagau caagggggcc 600

gguggugacu aagcggccgc ucgagcaugc aucuagagga uccccgggua ccgagcucga 660

auucaaaaaa aaaaaaaaaa aaaaaaaaaa aaaaaaaaaa aaaaaaaaaa aaaaaaaaaa 720

aaaaaaaaaa aaaaaaaaaa aaaaaaaaaa aaaaaaaaaa aaaaaaaaaa aaaaaaaaaa 780

aaa 783

<210> 3
<211> 44
<212> RNA
<213> Artificial sequence

<220>
<223> synthetic RNA

<400> 3
ucuuugagca ccuccagaaa aaaaaaaucu uugagcaccu ccag 44

<210> 4
<211> 44
<212> RNA
<213> Artificial sequence

<220>
<223> synthetic RNA

<400> 4
gcggcagcca cuucuugaaa aaaaaaagcg gcagccacuu cuug 44

<210> 5
<211> 44
<212> RNA
<213> Artificial sequence

<220>
<223> synthetic RNA

<400> 5
acucuuuguc gccuucgaaa aaaaaaaacu cuuugucgcc uucg 44

<210> 6
<211> 44
<212> RNA
<213> Artificial sequence

<220>

<223>  synthetic RNA

<400>  6
cucggccaca gcgaugcaaa aaaaaaacuc ggccacagcg augc                    44


<210>  7
<211>  44
<212>  RNA
<213>  Artificial sequence

<220>
<223>  synthetic RNA

<400>  7
gugggacagg cagaucaaaa aaaaaaagug ggacaggcag auca                    44


<210>  8
<211>  44
<212>  RNA
<213>  Artificial sequence

<220>
<223>  synthetic RNA

<400>  8
gcagccagcu uuccgggaaa aaaaaaagca gccagcuuuc cggg                    44


<210>  9
<211>  44
<212>  RNA
<213>  Artificial sequence

<220>
<223>  synthetic RNA

<400>  9
uugaagucuu cguuguuaaa aaaaaaauug aagucuucgu uguu                    44


<210>  10
<211>  44
<212>  RNA
<213>  Artificial sequence

<220>
<223>  synthetic RNA

<400>  10
cucuagaugc augcucgaaa aaaaaaacuc uagaugcaug cucg                    44


<210>  11
<211>  22
<212>  RNA
<213>  Artificial sequence

<220>
<223>  synthetic RNA

<400>  11

ucuuugagca ccuccagaaa aa                                                22

<210>  12
<211>  22
<212>  RNA
<213>  Artificial sequence

<220>
<223>  synthetic RNA

<400>  12
gcggcagcca cuucuugaaa aa                                                22

<210>  13
<211>  22
<212>  RNA
<213>  Artificial sequence

<220>
<223>  synthetic RNA

<400>  13
acucuuuguc gccuucgaaa aa                                                22

<210>  14
<211>  22
<212>  RNA
<213>  Artificial sequence

<220>
<223>  synthetic RNA

<400>  14
cucggccaca gcgaugcaaa aa                                                22

<210>  15
<211>  22
<212>  RNA
<213>  Artificial sequence

<220>
<223>  synthetic RNA

<400>  15
gugggacagg cagaucaaaa aa                                                22

<210>  16
<211>  22
<212>  RNA
<213>  Artificial sequence

<220>
<223>  synthetic RNA

<400>  16
gcagccagcu uuccgggaaa aa                                                22

<210>  17
<211>  22
<212>  RNA
<213>  Artificial sequence

<220>
<223>  synthetic RNA

<400>  17
uugaagucuu cguuguuaaa aa                                                22


<210>  18
<211>  22
<212>  RNA
<213>  Artificial sequence

<220>
<223>  synthetic RNA

<400>  18
cucuagaugc augcucgaaa aa                                                22


<210>  19
<211>  20
<212>  DNA
<213>  Artificial sequence

<220>
<223>  synthetic DNA

<400>  19
tgagattcct gggttcaagg                                                   20


<210>  20
<211>  20
<212>  DNA
<213>  Artificial sequence

<220>
<223>  synthetic DNA

<400>  20
gtcagaacac tgcacgttgg                                                   20


<210>  21
<211>  720
<212>  DNA
<213>  Artificial sequence

<220>
<223>  synthetic DNA

<400>  21
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtaa 720

<210> 22
<211> 44
<212> RNA
<213> Artificial sequence

<220>
<223> synthetic RNA

<400> 22
caccccggug aacagcuaaa aaaaaaacac cccggugaac agcu 44

<210> 23
<211> 44
<212> RNA
<213> Artificial sequence

<220>
<223> synthetic RNA

<400> 23
cgcugaacuu guggccgaaa aaaaaaacgc ugaacuugug gccg 44

<210> 24
<211> 44
<212> RNA
<213> Artificial sequence

<220>
<223> synthetic RNA

<400> 24
cuugccggug gugcagaaaa aaaaaaacuu gccgguggug caga 44

<210> 25
<211> 44
<212> RNA
<213> Artificial sequence

<220>
<223> synthetic RNA

<400>  25
guggucgggg uagcggcaaa aaaaaaagug gucgggguag cggc                        44


<210>  26
<211>  44
<212>  RNA
<213>  Artificial sequence

<220>
<223>  synthetic RNA

<400>  26
gcgcgggucu uguaguuaaa aaaaaaagcg cgggucuugu aguu                        44


<210>  27
<211>  44
<212>  RNA
<213>  Artificial sequence

<220>
<223>  synthetic RNA

<400>  27
gccaugauau agacguuaaa aaaaaaagcc augauauaga cguu                        44


<210>  28
<211>  44
<212>  RNA
<213>  Artificial sequence

<220>
<223>  synthetic RNA

<400>  28
cucgauguug uggcggaaaa aaaaaaacuc gauguugugg cgga                        44


<210>  29
<211>  44
<212>  RNA
<213>  Artificial sequence

<220>
<223>  synthetic RNA

<400>  29
cuucucguug gggucuuaaa aaaaaaacuu cucguugggg ucuu                        44


<210>  30
<211>  20
<212>  DNA
<213>  Artificial sequence

<220>
<223>  synthetic DNA

<400>  30
agaacggcat caaggtgaac                                                   20

<210>  31
<211>  20
<212>  DNA
<213>  Artificial sequence

<220>
<223>  synthetic DNA

<400>  31
tgctcaggta gtggttgtcg                                                    20